# EUROPÄISCHE PATENTSCHRIFT

(11) **EP 1 613 388 B1**
(45) Veröffentlichungstag und Bekanntmachung des Hinweises auf die Patenterteilung: **13.02.2008**
(21) Anmeldenummer: 05716301.6
(22) Anmeldetag: 22.03.2005
(51) Int. Cl.: A61M 27/00

(54) **EINSTELLBARES HYDROCEPHALUS-VENTIL**
ADJUSTABLE HYDROCEPHALUS VALVE
SOUPAPE REGLABLE POUR PATIENT ATTEINT D'HYDROCEPHALIE

(30) Priorität: 27.03.2004 DE 102004015500
(43) Veröffentlichungstag der Anmeldung: 11.01.2006
(73) Patentinhaber: Christoph Miethke GmbH & Co. KG, 14469 Potsdam (DE)
(72) Erfinder: MIETHKE, Christoph, 14558 Berlin-Nuthetal (DE)
(74) Vertreter: Kaewert, Klaus
(86) Internationale Anmeldenummer: PCT/EP2005/003052
(87) Internationale Veröffentlichungsnummer: WO 2005/092424

(56) Entgegenhaltungen:
- EP-A- 0 617 975
- DE-A1- 4 401 422
- DE-A1- 19 535 637
- FR-A- 2 768 057

## Beschreibung

Die Erfindung betrifft ein einstellbares Hydrocephalus-Ventil zum Druckausgleich des Liquor im Schädel eines Hydrocephalus-Patienten.

Hydrocephalus-Patienten haben folgendes medizinisches Problem:
Das Gehirn ist im Schädel von einer besonderen Flüssigkeit, dem Liquor, umgeben. Dieser Liquor wird ständig produziert und im gleichen Maße resorbiert. Bei der Erkrankung des Hydrocephalus, auch Wasserkopf genannt, ist dieses Gleichgewicht gestört, und es wird mehr Flüssigkeit erzeugt als abgebaut. Da der Schädelinnenraum ein geschlossenes Gefäß darstellt, kommt es zu einer Volumenvergrößerung. Beim Säugling können die Schädelnähte nicht zusammenwachsen, beim Erwachsenen steigt der Schädelinnendruck. Es gibt also einen Alters- und Kinderhydrocephalus.
Die Behandlung des Hydrocephalus erfolgt ursprünglich durch die bloße Ableitung des Liquors. Dies geschah durch die bloße Schlauchverbindung zwischen dem Schädel und einem großen venösen Blutgefäß oder durch eine entsprechende Verbindung des Schädels über einen Schlauch mit dem Bauchraum. Bald erkannte man jedoch, dass der Druck im Schädel einen bestimmten physiologischen Wert besitzen muss, wenn nicht wieder andere Komplikationen auftreten sollen.

Bei der Behandlung des Hydrocephalus wird mittels einer implantierbaren Drainage eine künstliche Verbindung hergestellt zwischen den Himkammern im Kopf und einem Ableitungskompartiment, heute meistens dem Bauchraum.

Es sind verschiedene Ventile bekannt, die in die Drainage für den Liquor eingebaut werden und mit deren Hilfe der Druck des Liquors eingestellt wird. Derartige Ventile werden im Bereich des Kopfes unter der Haut implantiert. Die Ventile sollen sich bei einem bestimmten kritischen Druck öffnen und den Abfluss von Liquor freigeben. Über eine Leitung - ebenfalls unter der Haut implantiert - wird der Liquor in die obere Hohlvene oder die Bauchhöhle abgeleitet.

Die bekannten Ventile helfen zwar bereits erheblich. Eine befriedigende Lösung ist mit den bekannten Ventilen jedoch noch nicht erreicht.

So sind Ventilentwicklungen bekannt, die eine perkutane, nicht invasive Einstellung des Öffnungsdruckes erlauben.
Dabei handelt es sich um Ventile, die dem Patienten implantiert werden und vorzugsweise über eine gleichfalls implantierte Schlauchleitung den überschüssigen Liquor aus dem Schädel des Patienten abziehen und vorzugsweise in eine Hohlvene oder in den Bauchraum ableiten. Dabei wird der Ventildruck durch eine Feder bestimmt, wobei die Feder über eine Mechanik verstellt wird, die ein schwenkbewegliches oder drehbewegliches Teil besitzt, das von außen durch Schwenken oder Drehen eines integrierten Magneten bewegt wird, so dass die Feder gespannt oder entlastet wird.
Die bekannten Ventile besitzen eine abgeflachte Bauweise. Ziel der flachen Bauweise ist es, beim Implantieren möglichst Beulen am Kopf des Patienten zu verhindern.

Zu den bekannten Ventilen gehört zum Beispiel das Codman Medos Ventil (Patent-Nr. US 5.928.182).
Dieses Ventil ist ein Kugelventil mit einer federbelasteten Kugel. Die Ventileinstellung erfolgt durch Änderung der Federstellung. Die Feder drückt mit dem einen Ende auf die Ventilkugel. Mit dem anderen Ende stützt sich die Feder auf einem Widerlager ab. Das Widerlager ist bei dieser Bauart durch Drehung höhenverstellbar. Die Höhenverstellbarkeit wird dadurch erreicht, dass das Widerlager schwenkbeweglich angeordnet ist und oben mit einem schräg verlaufenden Rand versehen ist, auf dem die Feder gleitet.

In der Patentanmeldung EP 1380317 A1 wird für dieses Ventil eine Verbesserung vorgeschlagen, durch die eine ungewollte Verstellung des Rotors und resultierend des Öffnungsdruckes verhindert werden soll. Hier greift eine in die Silikonummantelung integrierte Lasche seitlich am Rotor an, um eine Klemmung des Rotors seitlich zu bewirken und die nicht intendierte Verstellung des Ventils zu verhindern. Die Funktion einer solchen Konstruktion ist allerdings anzuzweifeln, da im Einsatzfall das Gehäuse mit einer wässrigen Lösung (Liquor) durchspült wird. Dadurch werden die Gleiteigenschaften zwischen Silikon und dem Rotor ungünstig verändert. Die elastische Spannung reicht für eine reibschlüssige Verdrehsicherung nicht aus.

Zu den bekannten Ventilen gehört auch das Sophysa-Ventil (Patent-Nr. FR 8105389; EP 0060369; US 4.443.214).
Selbst der Hersteller dieses Ventils äußert Bedenken, wenn der Hydrocephalus-Patient mit Permanentmagneten in Spielzeugen, Kopfhörern, Lautsprechern und elektro-magnetischen Feldern in Berührung kommt, wie sie von elektrischen Motoren, von Rasierern, Haartrocknern, Schaltern usw. ausgehen. Diese Warnung des Herstellers beinhaltet eine Warnung vor den meisten Lebensbereichen der Menschen. Da sich ein Mensch der zeitgemäßen Belastung mit solchen Dingen nicht entziehen kann, sind solche Lösungsvorschläge nicht praktikabel. Eine Verbesserung dieses Ventiltypes ist durch den Hersteller kürzlich auf den Markt gekommen (US 5.643.194). Hier gelingt in der Tat eine Sicherung gegen ungewollte Verstellung. Zwei auf einem drehbaren Rotor sitzende radial bewegliche Magnete sind so angeordnet, dass sie sich ohne Einwirkung von außen anziehen und dadurch den Rotor in am Gehäuse angebrachte Raster versperren. Von außen angebrachte stärkere Magnete können nun ventilseitig die Anziehungskräfte überwinden und die Magnete radial auseinanderziehen. Hierdurch wird der Rotor entsperrt, eine Verdrehung des Rotors durch Verdrehen der außen anliegenden Magnete um die Rotorachse ermöglicht eine Verstellung des Öffnungsdruckes. Dieses Prinzip hat sich als in der Praxis anfällig erwiesen. Zwar gelingt die Verhinderung einer ungewollten Verdrehung; die intendierte Verstellung gelingt aber häufig nicht. Das liegt an der Schwierigkeit, die genaue Position des Ventils sicher auszumachen, an den im Anwendungsfall schwer kalkulierbaren Reibungen, die innerhalb des Ventils eine Bewegung der Magnetschlitten erschweren oder sogar verhindern können. Nachteilig ist auch, dass hier durch dickere Hautschichten beide Magnetschlitten weit auseinander gezogen werden müssen, wobei die aus dem von außen angelegten Magnetfeld resultierenden Kräfte mit dem Abstand exponentiell kleiner werden und dann irgendwann nicht mehr ausreichen, um die Magnete auseinander zu ziehen. Eine Vereinfachung und Verbesserung ist hier notwendig und wünschenswert.

Das schon 1987 in der Patentschrift US 4.676.772 offenbarte Prinzip einer magnetischen Verklemmung eines drehbar angeordneten Rotors hat nie Marktreife erlangt. Auch hier zeigt sich offensichtlich die äußerst problematische Technik einer rein magnetisch realisierten Bremse in einem Hydrocephalus-Ventil. Durch extrem kleine Magnete, die in eine drehbare flache Scheibe integriert sind, an die zwei Stifte parallel zur Drehachse angeordnet sind, die in ein entlang eines Gewindeganges drehbares eine Kugel enthaltendes Schraubteil eintauchen, soll eine Höhenverstellung des Schraubteiles ermöglicht werden, wodurch die Kraft, die durch eine Silikonmembrane gegen die Kugel ausgeübt wird, gesenkt oder erhöht werden kann. Zur Verdrehung der Verstellscheibe muss zunächst durch von außen anliegende starke Magnete die anziehende Wirkung zwischen den in die Scheibe integrierten Magneten und einem ferromagnetisch wirkenden Gehäuseteil überwunden werden. Dadurch hebt die Scheibe ein wenig ab und eine Verdrehung soll möglich werden. Dieses Prinzip hat sich weder bewährt noch als praxistauglich erwiesen. Insbesondere die vielen kleinen bewegten Teile führen zu einer kritischen Addition von störenden Reibkräften, die insbesondere auf Grund der kleinen Baugröße vom Ventil und in der Folge auch von den verwendeten Magneten nicht beherrscht werden können. Die Stifte können sich sehr leicht verkanten, die außen mit einem großen Hebelarm angreifende Reibung im Gewindegang kann durch das aus den kleinen Magneten abgeleitete, sehr schwache magnetische Feld kaum überwunden werden, das Gewinde muss ein Spiel haben, um leichtgängig zu sein, was wiederum der Einstellgenauigkeit entgegenwirkt. Das Auffinden des Ventils ist auch hier problematisch, die präzise Verstellung nahezu unmöglich wegen der Kleinheit der zu bewegenden Teile. Außerdem ist wegen der Verwendung einer Silikonmembrane die wiederholgenaue Einstellung des Öffnungsdruckes nicht möglich, da sich die Materialbeschaffenheit unkalkulierbar ändert. Diese Konstruktion hat bis heute aus den genannten Gründen die Marktreife nicht erreichen können.

Ein weiteres System wird von der Firma Medtronic / PS-Medical angeboten (US 5.637.083) Auch hier wird vorgeschlagen, durch Verdrehung eines Rotors, in den zwei Magnete integriert sind, die Öffnungscharakteristik eines Ventils durch die Haut zu manipulieren. In dem Patent wurde vorgeschlagen, durch einen mechanischen, perkutan zu deaktivierenden Bolzen eine ungewollte Verstellung zu unterdrücken. Der klinische Einsatz hat jedoch offen gelegt, dass es durch den Bolzen zu einer kritischen Verklemmung des Rotors kommen konnte. Der Hersteller musste diese anfällige Technik vom Markt nehmen und fortan das Ventil ohne Verstellsicherung vermarkten. Die Gefahr der ungewollten Verstellung bleibt demnach virulent, insbesondere unter Einbeziehung der Forderung nach einer dem Bedarf geschuldeten sicheren Verstellung des Ventils.
Als weiterer Nachteil der bekannten Systeme hat sich erwiesen, dass die Verstellung immer sowohl in der Stehend- als auch in der Liegendposition wirksam ist. Dies ist aber häufig gerade unerwünscht. Vielmehr ist die gezielte Einstellung eines charakteristischen Ventildruckes, der nur in der stehenden Haltung des Patienten wirksam wird, therapeutisch sinnvoll und hilfreich. Auf Grund der nur in der stehenden Körperhaltung auftretenden hydrostatischen Druckdifferenz - insbesondere bei der ventriculo-peritonealen Ableitung - wird mit den bisher erhältlichen Systemen, wie sie oben beschrieben sind, bei Komplikationen in Folge von Überdrainage durch Hochstellen des Öffnungsdruckes zwar der zu hohen Drainage entgegen gewirkt, gleichzeitig aber eine gewollte, angemessene Drainage von Hirnwasser in der Liegendposition des Patienten - wie sie durch eine niedrige Einstellung des Öffnungsdruckes sichergestellt werden könnte - systematisch verhindert.
Die Konsequenz für den Patienten ist eine unphysiologische negative Druckbeaufschlagung des Hirnkammersystems unmittelbar nach dem Aufrichten. Dies kann zu Unwohlsein und Schwindel führen oder auch schwerwiegende Komplikationen wie subdurale Ergussbildung und Einblutungen verursachen, die operativ behandelt werden müssen. Mit den oben beschriebenen Ventilen kann man die auftretenden Komplikationen nicht vermeiden. Zwar kann durch Hochstellen des Ventils der negative Druck in der Stehendposition zum positiven hin verändert werden, gleichzeitig wirkt aber der hochgestellte Druck auch in der Liegendposition, wo er vollständig falsch ist. Das Hochstellen vermindert also einerseits den gefürchteten extrem negativen Druck im Stehen, verhindert aber gleichzeitig einen therapeutischen Effekt in der Liegendposition, insbesondere während der Ruhephasen der Patienten.

Der Erfindung liegt die Aufgabe zu Grunde, diese Nachteile von bisher bekannten verstellbaren Ventilen zu beseitigen. Das wird mit den Merkmalen der geltenden Patentansprüche erreicht. Die Erfindung widmet sich insbesondere der Aufgabe, nur in der Stehendstellung des Patienten sowie in partiellen Schräglagen eine Ventileinstellung zu ermöglichen, die auf die Einstellung der liegenden Position keinen Einfluß hat.

Dabei ist ein übliches gravitationsbetätigtes Ventil der Ausgangspunkt der Entwicklung , wie es zum Beispiel in dem Patent DE 4401422, Christoph Miethke, beschrieben ist. Gravitationsventile nutzen die Scherkraft einer oder mehrerer metallischer Kugeln vorzugsweise aus Tantal, um der in der Drainageleitung lagebedingt auftretenden Druckdifferenz einen von der Körperhaltung des Patienten abhängigen Gegendruck zur Vermeidung einer ungewollten Überdrainage entgegen zu stellen. Als Schließteil besitzen solche Ventile üblicherweise eine Kugel. Auch andere Schließteile kommen vor. In der Stehendstellung kommt der volle Gravitationsdruck zum Tragen. In der Liegendstellung kommt kein Gravitationsdruck zur Wirkung. In den Zwischenstellungen kommt der Gravitationsdruck teilweise zur Wirkung.

Aus dem Patent DE 19535637, Christoph Miethke, ist eine andere Bauart eines Gravitationsventiles bekannt, das aber hinsichtlich der Erfindung nicht über den Inhalt der DE 4401422 hinausgeht.

Nach der Erfindung wird der Gravitationsdruck des Ventils mit einer einstellbaren Federmechanik in der Stehendstellung ganz oder teilweise aufgehoben. Dieses Prinzip kann auf verschiedene Weise umgesetzt werden. Vorzugsweise wird das bewegliche Schließtei81 mit einem beweglichen und einstellbaren Gravitationsteil kombiniert, das eine einstellbare Belastung für das Schließteil bildet. Dadurch ergibt sich eine vorteilhafte Gestaltungsfreiheit. Es kann eine optimale Gewichtsverteilung zwichen dem Schließteil und dem Gravitationsteil vorgenommen werden. In der Regelung liegt das Optimum bei einem geringen Schließteilgewicht und einem vielfach höheren Gewicht des Gravitationsteils.

Die Form des Schließteils kann verschieden sein. In bisher bekannt gewordenen Lösungen sind ausschließlich Kugeln verwendet worden. Die Kugel bildet ein sehr bewährtes Schließteil. Das Gravitationsteil kann aber auch verschiedene andere Formen haben. Erfindungsgemäß eignet sich insbesondere auch ein Ring, eine Scheibe oder eine Glocke.

Wahlweise wird das Gravitationsteil durch eine zweite Kugel gebildet. Vorzugsweise ist die das Gravitationsteil bildende Kugel um einiges größer als die das Schließteil bildende Kugel. Dadurch erhöht sich das Gewicht der das Gravitationsteil bildenden Kugel. Zusätzlich kann das Gewicht des Gravitationsteiles noch durch Wahl eines Materials mit höherem Raumgewicht beeinflusst werden.

In der Ausführungsform mit einer zweiten, das Gravitationsteil bildenden Kugel sind die Kugeln in der Stehendposition übereinander angeordnet.
Vorzugsweise besteht die obere Kugel aus einem schweren metallischen Werkstoff mit hohem spezifischem Gewicht (besonders günstig ist hier die Verwendung von Tantal), die untere Kugel besteht aus einem möglichst leichten keramischen Werkstoff, wodurch durch die Gewichtskraft nur dieser Kugel ein nur sehr minimaler Öffnungsdruck von typischerweise 1 cm WS entsteht. Aber auch Titan hat gegenüber Tantal ein vergleichsweise geringes Gewicht.
Nach der Erfindung greift vorzugsweise eine Feder an die obere Kugel. Die Feder kann eine Blattfeder sein, die zwischen die Kugeln greift. Die Feder kann so eingestellt werden, dass in der Stehendposition das Gewicht der oberen Kugel ganz oder teilweise von der Blattfeder aufgefangen wird. Durch Verstellung der Feder wird das verändert.

Bei Erreichen der Liegendstellung verlieren beide Kugeln ihre Gravitationswirkung. Beide Kugeln geben die Ventilöffnung frei.

Mit der Verstellung kann das Ventil sehr leicht veränderten Bedingungen angepasst werden, die sich durch Wachstum, Dickleibigkeit oder aus sonstigen, mitunter auch therapeutischen Gründen ergeben.

Die beiden Kugeln befinden sich wahlweise in einer Führung, die durch zylindrische Bohrungen gebildet wird. In der Führung befindet sich eine ausreichende Öffnung für die Blattfeder.

Wahlweise hat das Gravitationsteil die Form einer Scheibe oder eines Ringes oder einer Kappe oder einer Glocke. Mit dieser Bauform eröffnen sich weitere Gestaltungsmöglichkeiten für das Gravitationsteil. Die Scheibe kann die größte Ausdehnung des Ventilgehäuses nutzen. Bei flacher Scheibenform des Ventils hat das Ventilgehäuse seine größte Ausdehnung entlang der Flachseite, eine gewünschte flache Bauweise wird möglich.
Nach der Erfindung kann die Scheibe trotz geringer Dicke auf Grund ihrer großen Ausdehnung leicht das gewünschte Gewicht besitzen. Ähnliches gilt für eine ringförmige Ausbildung des Gravitationsteiles. Der Ring kann andere Ventilteile umschließen und so zu einer kompakten Bauform führen.
Wahlweise werden die Vorteile der Scheibe und des Ringes in einer Kappenform oder Glockenform kombiniert.
Die Kappenform hat vorzugsweise einen zylindrischen Mantel und einen flachen scheibenförmigen Deckel. Die Glockenform hat wahlweise einen konisch verlaufenden Mantel. Als Werkstoff eignet sich Titan, bei kompakter Bauweise ist Tantal der ideale Werkstoff.
Das erfindungsgemäß als Scheibe oder als Ring oder als Kappe oder als Glocke ausgebildete Gravitationsteil ist vorzugsweise schwenkbeweglich im Ventilgehäuse angeordnet. Die schwenkbewegliche Anordnung bildet eine vorteilhafte Führung für das Gravitationsteil, da eine minimale Reibung bei den durch Lagewechsel induzierten Bewegungen des Gravitationsteils realisiert werden kann, was einer präzisen Arbeitsweise des Ventils entgegen kommt. Vorzugsweise ist die Lage der Schwenkachse so gewählt, dass das Gravitationsteil mittig auf die das Schließteil bildende Ventilkugel drückt. In der Stehendlage drückt das Gravitationsteil vorzugsweise genau vertikal auf den höchsten Punkt der das Schließteil bildenden Ventilkugel. Bei der erfindungsgemäßen Schwenkbewegung des Gravitationsteiles soll sich das Gravitationsteil möglichst wenig von der Vertikalen entfernen. Dazu wird die Schwenkachse so angeordnet, dass sie in der Stehendlage einen möglichst großen Abstand von der durch den höchsten Punkt der als Schließteil verwendeten Kugel gehenden Vertikalen hat.
Die Schwenkachse kann mit dem Gravitationsteil fest verbunden sein und schwenkbeweglich in dem Ventilgehäuse gelagert sein. Entsprechend dem erfindungsgemäßen Ausführungsbeispiel ist gerade die Fixierung des schwenkbaren Gravitationsteils an der Achse vorteilhaft

Die Ventilverstellung erfolgt mit extremer Sicherheit. Der eine Weg beinhaltet eine besondere Verstellung der Feder. Dabei ist ein besonders großer Verstellweg vorgesehen, der in eine Änderung der Federbelastung umgesetzt wird. Das heißt, bei vergleichbarem Änderungsbereich der Federbelastung ist ein größerer Verstellweg vorgesehen. In dem Umfang, in dem der Verstellweg größer wird, verringert sich die oben wiedergegebene Gefahr unerwünschter Verstellung.

Vorteilhafterweise erhöht sich zugleich die Genauigkeit der Verstellung mit größer werdendem Verstellweg.

Der andere Weg zur größeren Sicherheit wird von der Erfindung durch eine magnetbetätigte, in das Gehäuse integrierte Tellerfeder realisiert, die den Rotor verriegelt und eine ungewollte Verstellung verhindert.

Die Möglichkeit zur größeren Gestaltung des Verstellweges ergibt sich durch Änderung der Lage der Feder. Nach der Erfindung wird die Feder so gelegt, dass die Bewegungsebene der Feder bei deren Verstellung parallel zur Ebene liegt, in der die Schwenkbewegung oder Drehbewegung der Feder stattfindet. Im erfindungsgemäßen Sinne ist die Parallelität auch dann gegeben, wenn die Ebenen zusammenfallen.
Durch die erfindungsgemäße Anordnung der Feder kann die Feder sich in der Richtung bewegen, in der sich das Ventilgehäuse am weitesten ausdehnt. Das ist die Richtung der Flachseite.

Vorzugsweise findet als Feder ein Federstab Anwendung. Der Federstab kann unterschiedlichen Querschnitt besitzen, z. B. einen kleinen, runden Querschnitt. Dann wird von einer Drahtfeder gesprochen. Es kann auch ein flacher, breiter Querschnitt vorkommen. Dann wird von einer Blattfeder gesprochen.
Der Federstab ist schwenkbeweglich angeordnet und wirkt als ein Hebelarm. Vorzugsweise findet ein einarmiger Hebel Anwendung, dessen eines Ende gelenkig im Ventilgehäuse gelagert ist und dessen anderes Ende zwischen die oben erläuterten Kugeln greift. Die Federverstellung greift zwischen beiden Enden an der Feder.
Wahlweise bildet die Feder auch einen doppelarmigen Hebel, dessen einer Arm /Ende länger als der andere Arm / Ende ist. Nach Wahl steht das kürzere oder das längere Ende mit den Ventilkugeln in Eingriff. Das jeweils andere Ende wirkt mit der beschriebenen Verstellmechanik zusammen. Dabei ist eine gleitende, an sich bekannte Wirkverbindung zwischen der Feder und der drehbeweglichen oder schwenkbeweglichen Verstellmechanik vorgesehen. Das heißt, die Feder gleitet auf einer Fläche des drehbeweglichen oder schwenkbeweglichen Teils der Verstellmechanik.

Die Wirkverbindung mit dem als Kugel oder Klappe ausgebildeten Schließteiles wird dadurch gebildet, dass das kürzere Ende gleitend gegen das Schließteil drückt.
Die Wirkverbindung mit der Verstellmechanik hängt von der Gestaltung der Verstellmechanik ab. Vorzugsweise ist als Verstellmechanik eine Dreheinrichtung oder Schwenkeinrichtung vorgesehen, die mit einer Gleitfläche versehen ist bzw. mit einer Gleitfläche der Feder zusammenwirkt. Die Auswirkungen einer Verstellung sind von der Gestaltung der Feder abhängig.
Die Feder kann einen doppelarmigen Hebel bilden. Dann steht vorzugsweise das eine Federende mit dem Gravitationsteil in Wirkverbindung und steht das andere Ende mit der Verstelleinrichtung in Verbindung und ist die Feder dazwischen schwenkbeweglich im Ventilgehäuse gelagert.
Sofern die Feder einen doppelarmigen Hebel bildet und wenn die Hebelarme unterschiedliche Länge haben, so kommt es darauf an, wo die Verstelleinrichtung angreift.
Wenn die Verstelleinrichtung an dem kurzen Hebelarm angreift, so bewirkt die Verstellbewegung eine größere Verformung des langen Hebelarmes der Feder. Wenn die Verstelleinrichtung an dem langen Hebelarm angreift, so bewirkt die Verstellbewegung eine kleinere Verformung des kurzen Hebelarmes.
Vom Umfang der Verformung der Feder ist die Kraft abhängig, mit der die Feder das Schließteil in der Stehendstellung von dem Gewicht des Gravitationsteiles entlastet.

Wahlweise kann die Feder auch als einarmiger Hebel ausgestaltet sein. Dies entsteht, wenn die Feder an einem Ende mit dem Gravitationsteil in Wirkverbindung steht und an dem anderen Ende schwenkbeweglich im Ventilgehäuse gelagert ist und wenn die Verstelleinrichtung dazwischen an die Feder angreift.

Vorzugsweise ist die Verstelleinrichtung für eine Gleitverbindung mit der Feder an der Gleitfläche als Kurvenbahn ausgebildet.

Die Kurvenbahn läuft vorzugsweise zumindest teilweise spiralförmig an dem schwenkbeweglichen oder drehbeweglichen Teil. Der Umfangswinkel an dem schwenkbeweglichen bzw. drehbeweglichen Teil umfasst insbesondere mindestens 300 Grad.

Die Feder kann an der Kurvenbahn aufwärts oder abwärts gleiten. Die Bewegungsrichtung ergibt sich aus der Drehrichtung bzw. Schwenkrichtung des drehbeweglichen bzw. schwenkbeweglichen Teiles.
Wahlweise kann das drehbewegliche Teil auch in der gleichen Drehrichtung weiterbewegt werden und gleichwohl wieder an den Verstellanfang kommen. Das wird dadurch erreicht, dass zwischen dem Anfang der Kurvenbahn und dem Ende der Kurvenbahn an dem drehbeweglichen oder schwenkbeweglichen Teil eine Verbindung vorgesehen ist.

Die erfindungsgemäße Feder hat wahlweise eine Winkelform. Die beiden Hebelarme des doppelarmigen Hebels stehen in einem Winkel zueinander, der kleiner als 180 Grad ist, auch kleiner als 90 Grad sein kann.

Der Querschnitt der erfindungsgemäßen Feder kann beliebig sein. Günstig sind runde und rechteckige Formen. Besonders günstig ist eine Feder mit blattförmigem oder drahtförmigem Querschnitt.

Zur schwenkbeweglichen bzw. drehbeweglichen Lagerung der Feder eignet sich zum Beispiel ein Stift, dessen Enden in entsprechende Ausnehmungen im Ventilgehäuse bzw. im Ventildeckel greifen. Die Enden des Stiftes können auch spitz ausgebildet sein, so dass der Stift in den Ausnehmungen auf den Spitzen dreht. Diese Vorgehensweise ist technisch und wirtschaftlich günstig.

Zur Befestigung des Stiftes an der Feder eignet sich eine Schweiß- oder Lötverbindung, auch andere Verbindungen. Darüber hinaus kommen auch andere mechanische Verbindungen wie Klemmverbindungen und Steckverbindungen in Betracht.

Für die Funktion der erfindungsgemäßen Feder kann es günstig sein, wenn der lange Hebelarm an dem schwenkbeweglichen Teil bzw. drehbeweglichen Teil der Verstellmechanik geführt ist. Dazu kann dieses Teil zugleich an mindestens einer Seite die Feder führen. An der anderen Seite kann die Führung durch eine Scheibe gebildet werden.

Bei Verwendung einer Ventilkugel als Schließteil ist es ventilkugelseitig günstig, wenn eine große Fläche zur Berührung zwischen der Feder und der Ventilkugel zur Verfügung steht. Soweit die Feder diese großflächige Berührung nicht ermöglicht, kann an dem betreffenden Federende ein Blech befestigt werden. Das Blech ist wahlweise angeschweißt oder angelötet oder in sonstiger Weise befestigt.

Die Verstelleinrichtung wird in der jeweiligen Drehstellung gesichert. Dazu eignen sich unterschiedliche Systeme. Es können rein mechanisch wirkende Bremsen zur Anwendung kommen. Vorzugsweise findet eine selbst aktivierende Bremse Anwendung, die dadurch entsteht, dass die Verstelleinrichtung im Ventilgehäuse nach jeder Verstellbewegung reibungsschlüssig eingespannt wird. Für eine weitere Verstellung wird das Ventilgehäuse so verformt, dass das Ventilgehäuse mit seinen Reibungsflächen von den korrespondierenden Reibungsflächen der Verstelleinrichtung abhebt. Die notwendige Verformung entsteht durch Druck. Nach einem bekannten Vorschlag können die Reibungsflächen sich an den Seiten des Ventilgehäuses befinden. Nach einem älteren anderen Vorschlag befinden sich die Reibungsflächen an anderer Stelle, nämlich unmittelbar am Boden oder Deckel des Ventilgehäuses oder an einem mit dem Boden oder Deckel zusammenwirkenden Teil der Verstelleinrichtung. Durch Eindrücken des Boden oder Deckels wird die Verstelleinrichtung für eine Verstellung frei. Durch Loslassen des eingedrückten Bodens bzw. Deckels formt sich dieser zurück und es kommt zur reibungsschlüssigen Verbindung zwischen dem Deckel bzw. dem Boden und der Verstelleinrichtung.

Die Sicherung der Verstelleinrichtung in der jeweiligen Drehstellung kann auch nach einem anderen bekannten Konzept mit Hilfe von Magneten erreicht werden. Dabei können sich an der Verstelleinrichtung und / oder an der Gehäuseinnenseite Magnete / Dauermagnete befinden. Die Magnete bilden einen Teil einer Arretierungseinrichtung. Zu der Arretierungseinrichtung können andere bewegbare Teile gehören, die auf Grund der Magnetkraft in Arretierungslöcher oder in eine Verzahnung greifen. Durch Anwendung stärkerer Magnete kann die Arretierung jedoch gelöst werden.

Für die Anwendung der Magnete sind reaktive Materialen, zum Beispiel Stahl oder ebenfalls Magnete, von Vorteil.

Außerdem ist es zweckmäßig, die Reibungsflächen so zu wählen, dass eine besonders große Selbsthemmungswirkung entsteht. Dazu ist nach der Erfindung ein Mindestabstand der Reibungsflächen zur Drehachse bzw. Schwenkachse des schwenkbeweglichen bzw. drehbeweglichen Teiles vorgesehen. Vorzugsweise befinden sich die Reibungsflächen im größtmöglichen Abstand von der Ventilmitte am äußeren Rand des drehbeweglichen bzw. schwenkbeweglichen Teiles der Verstelleinrichtung.

Als Magnete finden vorzugsweise kleine Bauformen als Stiftmagnete gemäß den Patentansprüchen Verwendung. Die kleinen Magnete tragen auch zu geringen Ventilabmessungen bei, wie sie Gegenstand der Patentansprüche sind.

Die Verstelleinrichtung für das erfindungsgemäße Ventil kann gleichfalls mit extrem kleinen Abmessungen gestaltet werden. Nach der Erfindung wird das zur Reduzierung des Durchmessers der Verstelleinrichtung und zu einer besonderen Formgebung der Verstelleinrichtung genutzt, nämlich zur Gestaltung der Verstelleinrichtung in Stiftform, ähnlich einem Kugelschreiber. Das erlaubt die Handhabung der Verstelleinrichtung wie die Handhabung eines Stiftes oder Kugelschreibers. Solche Instrumente können praktischerweise in einer Brusttasche getragen werden. Zugleich wird die Mechanik eines Kugelschreibers genutzt, um die am Kopf des Instrumentes vorgesehenen Magnete zur Messung des eingestellten Druckes zu nutzen, oder aber gerade zur Einstellung des Druckes. Eine ähnliche Mechanik wie die im Kugelschreiber findet Verwendung, um die Mine ein- oder auszufahren. Das wird hier dazu genutzt, die Magnettrommel mit der Verstelleinheit reibschlüssig zu verbinden oder zu lösen. Ist die Verbindung gelöst, arbeitet das Instrument als Messstift, besteht die Verbindung, arbeitet es als Verstellstift.

Wahlweise besitzt die erfindungsgemäße stiftförmige Verstelleinrichtung am vorderen Ende eine Kappe, mit dem die Verstelleinrichtung aufgesetzt wird. Bei lockerem Aufsetzen der Verstelleinrichtung bewirken die Magnete selbsttätig eine Zentrierung Der Verstelleinrichtung, so dass es leicht ist, die Verstelleinrichtung durch Drehen zu betätigen.
Nach der Zentrierung der Verstelleinrichtung ist vorzugsweise eine elastische Verformung des Ventils vorgesehen. Die Verformung soll durhc kontrolliertes Andrücken der Verstelleinrichtung an das implantierte Ventil erfolgen. Die Verformung führt nach der Erfindung zu einem Abheben des schwenkbeweglichen bzw. drehbeweglichen Teiles von dem Ventilgehäuse. Entsprechend verringert sich die Reibung. Sie verringert sich auf ein Minimum, was als Aufhebung der Reibung bezeichnet wird.
Nach Aufhebung der Reibung kann das schwenkbewegliche bzw. drehbewegliche Teil ventilseitig leicht mit der Verstelleinrichtung bewegt werden. Bzw. dessen Position annehmen.

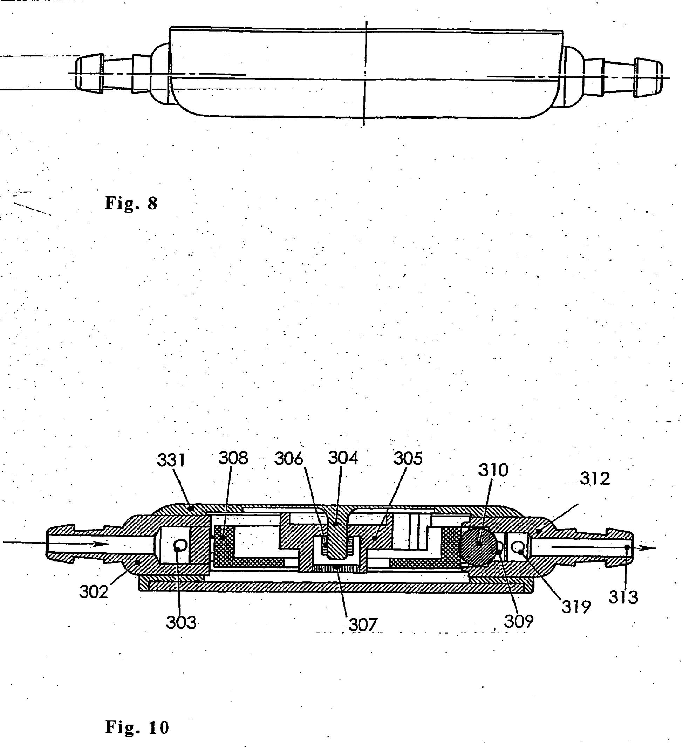

In der Zeichnung ist in den Fig. 8,9 und 10 ein Ausführungsbeispiel der Erfindung dargestellt.

Das Ventil besteht aus einem massiven Metallgehäuse 330. Zu dem Ventil gehören darüber hinaus im Ausführungsbeispiel eine Ventilkugel 310 als Schließteil des Ventils, ein Gravitationsteil 308 zur Erzeugung eines erhöhten Schließdruckes im Ventil, ein Federsystem 316 mit einer Verstelleinrichtung und einer Arretierungseinrichtung. Das Ventil befindet sich in einer Liquor-Leitung, die einem Hydrocephaluspatienten implantiert ist. Das Ventil soll in der Liegendstellung von dem überschüssigen Liquor durchströmt werden, während der Liquor-Abfluss in der Stehendlage des Patienten durch einen erhöhten Schließdruck des Ventils erschwert werden soll.

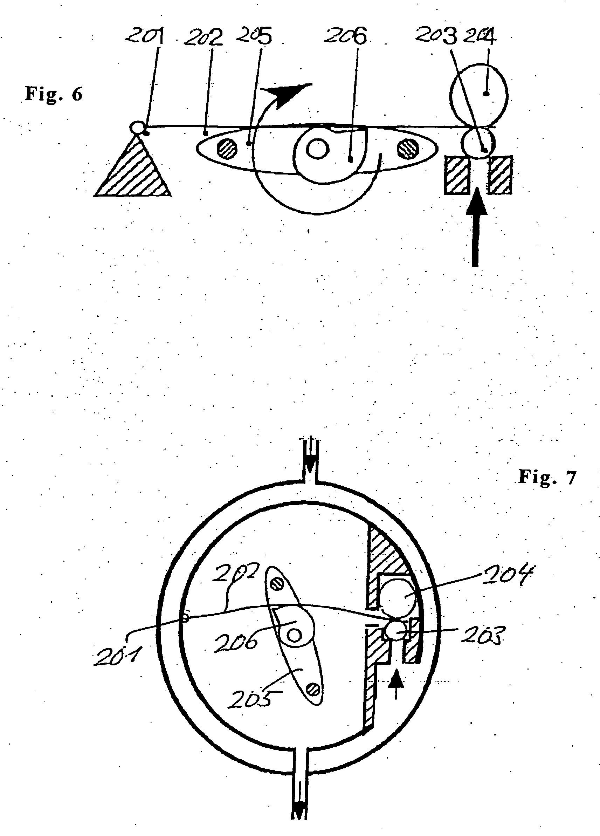

Fig. 6 zeigt ein Funktionsprinzip eines Schließteiles und eines Gravitationsteiles, zusammen mit einem Federsystem und einer Verstelleinrichtung.

In ein nicht in Fig. 6 dargestelltes Ventilgehäuse ist ein Gelenk 201 integriert, an dem eine schwenkbare Feder in Form einer Blattfeder 202 befestigt ist. Die dem Gelenk 201 gegenüberliegende Seite der Feder ruht auf einer Ventilkugel 203, die entweder unmittelbar Bestandteil des Ventilsitzes ist und aus Saphir oder Rubin oder einem anderen keramischen Werkstoff oder auch aus Titan besteht. Über der Kugel 203 ist eine weitere Kugel als Gravitationsteil des Ventils angeordnet. Es handelt sich um eine Metallkugel. Geeignetes Metall ist zum Beispiel Tantal oder Edelstahl. Grundsätzlich eignen sich alle biokompatiblen Materialien mit einem möglichst hohen spezifischen Gewicht. Die Feder 202 greift zwischen die beiden Kugeln 203 und 204. Zwischen beiden Auflagepunkten der schwenkbaren Feder 202 ist ein magnetisch aktivierbarer Rotor 205 angebracht. In der Mitte des Rotors befindet sich ein Exzenter 206, der fest mit dem Rotor verbunden ist. Die Verdrehung des Rotors ist durch externe Magnetfelder möglich. Die externen Magnetfelder können elektrisch erzeugt werden oder zu Permanentmagneten gehören.

Wird die Verstellung des Rotors vorgenommen, so führt dies zu einer Verformung der Blattfeder zwischen Drehlager und Kugelauflage. Hierdurch wird eine Federkraft erzeugt, die gegen die Schwerkraft der Gewichtskugel wirkt.

Fig. 7 zeigt eine schematische Darstellung eines Ventilgehäuses, in welches das System nach Fig. 6 integriert ist. Dabei ist auch der Liquorfluß durch das Gehäuse dargestellt. Der Liquor tritt oben ein und fließt durch einen Kanal in dem Ventilgehäuse gegen die Ventilkugel 203, die in der Stehendlage durch die Kugel 204 belastet ist.

Die in Fig. 7 dargestellte Ventilposition zeigt die Einstellung des minimalen Öffnungsdruckes. Bei dem Ventil wird der Öffnungsdruck bestimmt durch die Grafitationskraft der Kugel 204, den Öffnungsquerschnitt am Einlass unterhalb der Saphirkugel 203 sowie durch die eingestellte Federkraft, die der Gravitationskraft entgegenwirkt. In der dargestellten Ventilposition hat die Verstelleinrichtung die Maximalstellung erreicht. Das ist gleichbedeutend mit einer maximalen Verformung der Feder und einer maximalen Entlastung des Gravitationsteiles 204. Diese Federkraft könnte so ausgelegt werden, dass sie die Gravitationskraft bei maximaler Vorspannung vollständig kompensiert oder aber nur teilweise kompensiert.

Fig. 1 zeigt einen größeren Schnitt durch ein Gehäuse mit anderem Federsystem und anderer Verstelleinrichtung und mit einem größeren Detaillierungsgrad als in Fig. 10 zur Darstellung der Wirkungsweise der Bremse.

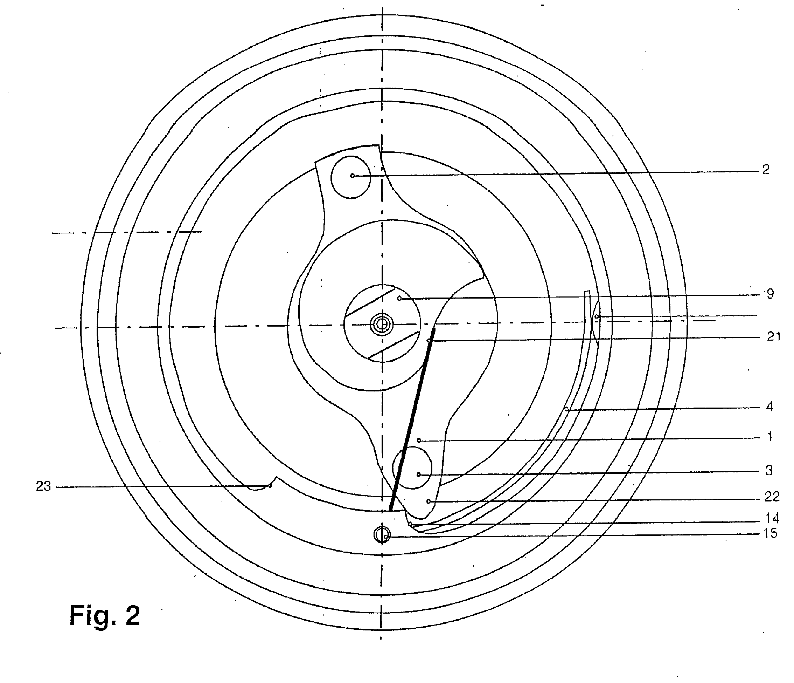

Das Ventil besteht aus einem massiven Titangehäuse 16. Das Ventilgehäuse 16 hat eine Ringform und wird beidseitig durch Deckel 18 und 20 verschlossen.
Fig. 2 zeigt einen anderen Schnitt durch das Gehäuse, so dass das Federsystem und die Verstelleinrichtung erkennbar sind. Zu dem Federsystem gehört eine Blattfeder 4.
Die Blattfeder 4 bildet mit dem Federdraht 3 und einer in Fig. 2 sichtbaren Achse 15 eine funktionale Einheit. In die als Rotor 1 bezeichnete Verstelleinrichtung sind zwei Magnete 2 und 3 mit umgekehrter Polung eingebracht. Der Rotor 1 ist auf einer Achse 7 gehalten. Die Achse 7 befindet sich am Deckel 18. Der Deckel 18 ist in Bezug auf das Ventil nach außen gewölbt. Der Rotor 1 ist mit einer Schraube 6 auf der Achse 7 so gegen den Deckel gespannt, dass durch die Anpressung des Rotors 1 am Deckel 18 eine reibungsschlüssige Verbindung des Rotors 1 mit dem Deckel 18 entsteht und der Rotor 1 an einer unbeabsichtigten Drehung gehindert wird. Der Reibungsschluss besteht so lange, bis eine unten erläuterte Verstellung erfolgt. Der Reibungsschluss ist so groß gewählt, dass keine unbeabsichtigte Rotorverstellung erfolgt, auch nicht durch unvorhergesehene Magnetfelder.
Der Reibungsschluss entsteht an den Außenkanten des Rotors 1. Das resultiert aus der nach außen gerichteten Krümmung des Deckels und daraus, dass die korrespondierende Rotorfläche eben ist.
Wenn auf den Deckel 18 gedrückt wird, so erfährt der Deckel eine elastische Verformung. Der Deckel wird flacher oder nimmt sogar eine gegenteilige Wölbung an. Dabei lösen sich die Außenkanten des Rotors von dem Deckel 18 und der Rotor kann mit Magnetkraft gedreht werden. Der Deckel 18 hat vorzugsweise eine Stärke von 0,1 bis 0,2 mm, in anderen Ausführungsbeispielen eine Stärke bis 0,5 mm. Die mit der elastischen Verbiegung verbundene Verformung beträgt vorzugsweise 0,01 mm bis etwa 0,1 mm, in anderen Ausführungsbeispielen bis zum zweifachen der Deckelstärke. Je stärker der Deckel vorgespannt ist, desto stärker muss später von außen gedrückt werden, um ein Abheben des Rotors 1 bei 6 von dem Deckel 18 zu bewirken und die Arretierung des Rotors 1 am Deckel 18 aufzuheben.

Die Stellung des Rotors 1 definiert die Federkraft, die der Gewichtskraft in der aufrechten Körperhaltung kompensierend entgegengestellt wird.

In Fig. 2 ist ein von unten geöffnetes Ventil dargestellt. Zu sehen ist hier die Feder 10, die verschweißt ist mit der Achse 15 und der Blattfeder 4. Diese Komponenten sind vorzugsweise aus einem metallischen Material hergestellt, insbesondere aus Titan oder einer Titanlegierung. Der Federdraht der Feder 10 hat vorzugsweise den Durchmesser 0,1 mm, in anderen Ausführungsbeispielen kann der Federdraht bei kürzeren Längen einen geringeren und bei größeren Längen einen größeren Querschnitt haben. Der Querschnitt des Federdrahtes ist im Ausführungsbeispiel kreisförmig. Die Blattfeder hat vorzugsweise eine Stärke von ebenfalls 0,1 mm und eine Höhe von etwa 1 mm. Für andere Ausführungsbeispiele mit kleineren Längen und mit größeren Längen gelten die Hinweise zum Draht der Feder 10 entsprechend. Die Blattfeder ist sehr steif.

Die Achse 7 besitzt in der Zeichnung links einen Absatz und Zapfen, mit dem sie in eine kleinere Bohrung des Teiles 9 am Rotor 1 ragt. Im Einbauzustand besteht zwischen der Achse 7 und dem Teil 9 ein Spalt an der Stelle 19. Der Spalt beträgt vorzugsweise 0,01 mm oder aber auch 0,1 mm oder noch mehr.

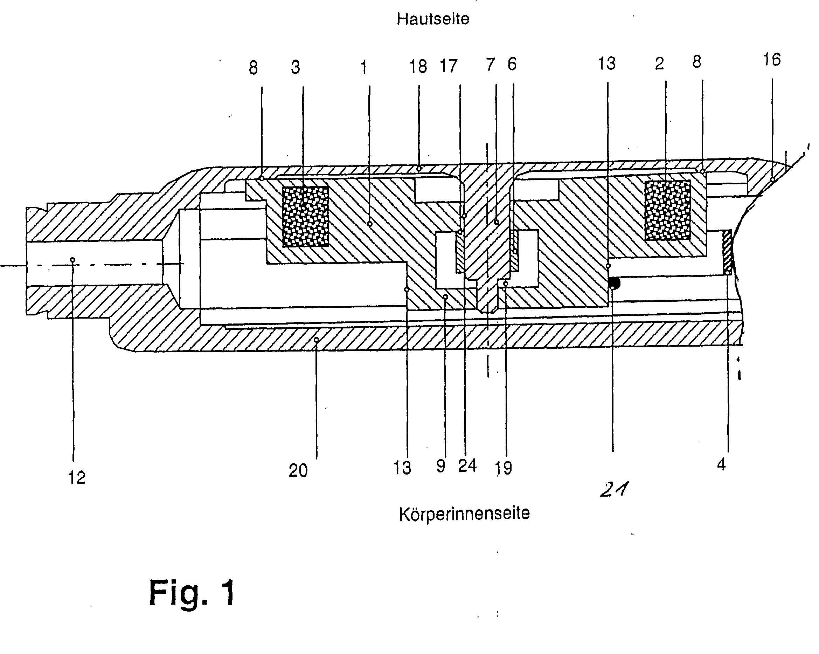

In Fig. 1 ist die Hautseite in der Zeichnung rechts und die Körperinnenseite in der Zeichnung unten dargestellt. Wird nun von außen durch die Haut mechanisch ein Druck auf den Deckel 18 ausgeübt, so wird dadurch in Abhängigkeit von der Kraft der Deckel 18 nach innen verformt / gewölbt und die Achse 7 nach unten zum Deckel 20 gedrückt. Hierdurch wird der Spalt 19 geschlossen, die Achse 7 drückt gegen Teil 9 und hebt somit den gesamten Rotor von dem Deckel 18 ab. Die elastische Vorspannung des Deckels 18 und die Reibkräfte an der Stelle 8 werden dabei aufgehoben. Jetzt entsteht an der Stelle 8 ein Spalt, der Rotor ist nun frei drehbar. Wird die äußere Last wieder weggenommen, geht der äußere Deckel 18 wieder in seine Ausgangslage zurück und erzeugt die elastische Vorspannung zwischen Auflagepunkt 17 und Auflagepunkt 8. Der Rotor wird wieder im Gehäuse geklemmt, eine Verdrehung ist nicht möglich.

Der Rotor 1 besitzt eine Kurvenscheibe 13.
In Fig. 2 ist der Rotor 1 in der Minimalposition dargestellt. Durch Verdrehung um etwa 300° wird die Feder 10 am Auflagepunkt 21 entsprechend der Kurvenscheibe 13 in ihre Maximalposition gebracht, so dass der resultierende Öffnungsdruck nun maximal wird. Der Höhenunterschied zwischen der minimalen und maximalen Federvorbiegung von Teil 4 oder 10 beträgt in etwa 0,7 bis 0,8 mm. Konkret richtet sich dies jedoch nach der Abmessung des gewählten Titandrahtes 10.
Die Anordnung der beiden Magnete 2 und 3 erfolgt so, dass ein außen anliegendes Magnetfeld ein maximales Drehmoment erzeugen kann.
D. h., der Abstand der beiden Magnete beträgt im Ausführungsbeispiel 7 mm, in einem anderen Ausführungsbeispiel 8 mm und in noch weiteren Ausführungsbeispielen bis 20 mm. Konkret richtet sich dieser Abstand nach den Außenabmessungen des Gehäuses. Das kreisrunde Gehäuse hat vorzugsweise einen Durchmesser von 14 mm, in anderen Ausführungsbeispielen bis 19 mm und in noch weiteren Ausführungsbeispielen bis 31 mm und ist ergonomisch geformt, so dass einerseits die Lage des Ventils von außen gut getastet werden kann, aber andererseits das über dem Ventil liegende Gewebe nicht geschädigt wird. Scharfe Kanten werden daher vermieden.

Der Rotor hat eine Spitze 22, in Fig. 2 dargestellt. Diese Spitze schlägt gegen Anschlag 14 beim minimalen Wert und gegen Anschlag 23 beim maximalen Wert. Durch diesen Anschlag-wird im Ausführungsbeispiel verhindert, dass Maximal- und Minimaleinstellungen unmittelbar ineinander übergehen und jederzeit gut unterscheidbar bleiben. In anderen Ausführungsbeispielen ist wahlweise ein Übergang vorgesehen.

Die Achse 15 hat vorzugsweise einen Durchmesser von 0,3 mm und kann ggf. oben und unten eine Spitze aufweisen, um die Lagerkräfte zu minimieren. Auf Grund der beschriebenen Bauweise ist eine Verdrehung des Rotors 1 nur möglich, wenn der Deckel 18 in der Zeichnung nach links gepresst und der Rotor 1 dadurch frei drehbar wird. Gleichzeitig muss in diesem Zustand ein spezifisches Magnetfeld von außen angeordnet werden, um eine Verdrehung sicher zu stellen. Wird der Deckel dann entlastet, wird die Position des Rotors durch elastische Klemmung fixiert. Entsteht nun zwischen dem Einlass und dem Auslass des Ventils ein Differenzdruck, der größer ist als der Öffnungsdruck des Ventils, wird die Kugel aus ihrem Ventilsitz gegen die Blattfeder gedrückt und das Ventil wird geöffnet. Dadurch wird es möglich, dass Hirnwasser vom Einlass zum Auslass das Ventil durchströmt und ein weiterer Druckanstieg verhindert wird. Die tatsächliche Ventilcharakteristik wird definiert durch die Verdrehposition des Rotors 1 bzw. die daraus resultierende Position des Auflagepunktes 21 auf der Spirale bzw. der Kurvenscheibe 13. Durch gezielte Änderung der Form der Kurve kann in anderen Ausführungsbeispielen auch ein nicht linearer Verlauf der Öffnungscharakteristik in Abhängigkeit vom Verdrehwinkel des Rotors 1 eingestellt werden. Vorzugsweise ist der Rotor so gefertigt, dass in allen Ausgangspositionen des Rotors eine Verdrehung von 10 Grad in die eine oder andere Richtung die jeweils gleiche Änderung des Öffnungsdruckes des Ventils nach sich zieht. Die Anordnung der Magnete 2 und 3 möglichst weit auseinander hat den Vorteil, dass mit möglichst geringen Magnetkräften möglichst große Verstellmomente realisiert werden können. Die hier verwendeten Neodym-Magnete haben eine zylindrische Form mit einem Durchmesser von 1 mm und einer Höhe von etwa 1,2 mm. Die Fertigung von Gehäuse und Rotor sowie die Fertigung der anderen Komponenten aus Titan hat den Vorteil, mit präzisen Passungen ein ideales Lagerspiel einstellen zu können und ungewolltes Spiel ebenso wie ungewollt erhöhte Reibung systematisch zu vermeiden. So hat die Achse 7 vorzugsweise einen Durchmesser von 1 mm, das Spiel an Position 24 zwischen Achse 7 und Rotor 1 ist vorzugsweise durch eine enge Spielpassung toleriert. Eine eben solche Spielpassung ist vorgesehen zur Lagerung der Achse 15 im Ventilgehäuse. Diese Achse 15 ist ähnlich einer Türangel im Ventilgehäuse gelagert und ermöglicht die nahezu reibungsfreie Verdrehung der Blattfeder 4 im Rahmen des Öffnens und Schließens des Ventils. Die Bauhöhe des Ventils liegt bei etwa 4,5 mm, wesentlich niedrigere Bauhöhen sind nicht unbedingt wünschenswert, wenngleich möglich, da das palpatorische Auffinden des Ventils nicht zu schwierig sein soll.

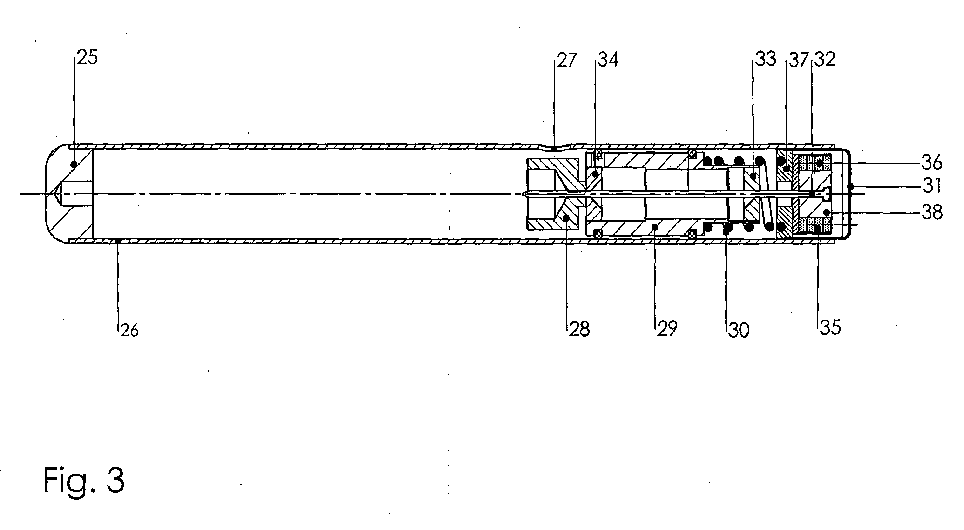

Für die Verstellung des Ventils sind spezielle Verstellstifte entwickelt worden. Ein Ausführungsbeispiel eines solchen Stiftes ist in Fig. 3 dargestellt. Die Darstellung beinhaltet auch eine Vergrößerung gegenüber dem Ausführungsbeispiel, jedoch weniger groß als in Fig. 1 und 2. Um auf die richtigen Maßrelationen des Ventils zum Stift als Verstelleinrichtung zu kommen, wird empfohlen, den Stift in entsprechender Vergrößerung zusammen mit dem Ventil zu betrachten.
In maßstabsgetreuer Darstellung sind alle Einzelheiten so klein, dass sie nicht erkennbar werden.

Ein dünnwandiges Röhrchen 26 mit einem Durchmesser von etwa 12 mm ist an einem Ende durch einen Stopfen 25 verschlossen. An der anderen Seite ist eine nadelgelagerte Messmechanik eingebaut. Dazu gehören: Messtrommel 28, auf deren Oberfläche eine Skalierung aufgebracht ist, die Messtrommel ist mit der Achse 32 verbunden, die in der Lagerbuchse 29 an den Stellen 34 und 33 gelagert ist. Die Lagerbuchse 29 ist so in das Röhrchen 26 eingebracht, dass eine Verschiebung und Verdrehung derselben nicht möglich ist. An der nicht verschlossenen Seite des Röhrchens ist eine bewegliche Kappe in das Röhrchen eingeführt, die durch eine Federkraft nach außen gedrückt wird. Die Feder 30 stützt sich an der Lagerbuchse 29 ab und drückt den Ring 37 gegen die Kappe 31. Mit der Nadel 32 verbunden ist der Zylinder 38.
In den Zylinder 38 eingebracht sind Magnete 35 und 36. Bei dem einen Magnet ist der außen liegende Pol negativ, bei dem anderen positiv. Der Abstand der Magnete entspricht in etwa dem Abstand der Magnete innerhalb des Ventils, ebenso der Durchmesser.

Durch die Kappe 31 und die Feder 30 ist ein Verdrehen der Achse des Rotors und des Zylinders 38 nicht möglich, solange nicht die Kappe gegen die Federkraft auf die Lagerbuchse-gedrückt wird. Erst wenn der Stift oberhalb des Ventils gegen den Kopf des Patienten gedrückt wird und somit die Kappe 31 in das Stiftgehäuse drückt, ist eine Drehung von Rotor und Skalentrommel sowie Magnetzylinder möglich. Der Stift ist so gegen den Kopf des Patienten zu drücken, dass das Fenster 27 um 90 Grad verdreht zur Körperachse eingesehen werden kann. Hierdurch wird sichergestellt, dass Ventilstift und das Ventil selbst die gleichsinnige Orientierung haben. Wird nun die Kappe oberhalb des Ventils vor den Kopf des Patienten gedrückt, folgt die Stellung des Rotors innerhalb des Stiftes der Stellung des Rotors innerhalb des Ventils, da der Ventilrotor durch die elastische Klemmung in seiner Position nicht verändert werden kann, der Stiftrotor jetzt aber wegen der feinen Nadellagerung an den Stellen 33 und 34 durch Verdrehung sich der Position des ventilseitigen Rotors anpassen kann. An dem Fenster 27 kann der entsprechende Einstellungsdruck des Ventils nun leicht ausgelesen werden. Diese Konstruktion gewährleistet ein sicheres, jederzeit leicht wiederholbares Messen. Durch die Fixierung des Messwertes nur wenige Zehntelmillimeter entfernt vom Kopf ist eine Verdrehung nach Wegnehmen des Stiftes vom Kopf des Patienten nicht mehr möglich. Das Messergebnis wird unmittelbar eingefroren.

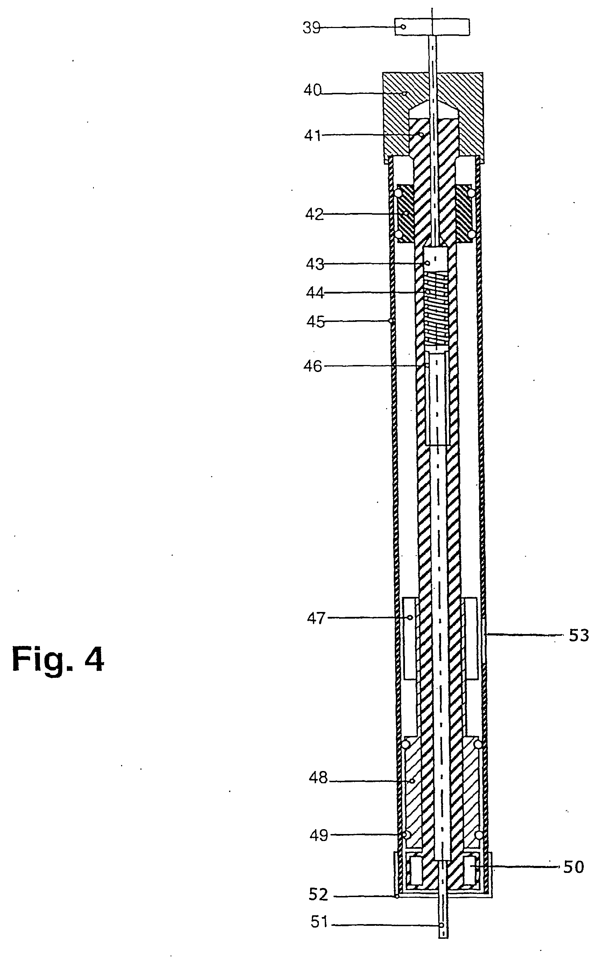

Fig. 4 zeigt ein anderes Ausführungsbeispiel eines Verstellstiftes. Die Abmaße entsprechen in etwa den Maßen eines gewöhnlichen Kugelschreibers, d. h., das Röhrchen hat einen Außendurchmesser von vorzugsweise 12 mm und eine Länge von etwa 10 cm. Das Einstellrad 40 ist fest auf der Achse 41 fixiert. Eine Verdrehung dieses Rades bewirkt eine Verdrehung der Achse. Am unteren Ende der Achse 41 sind in die Achse zwei Magnete 50 zylinderförmig eingebracht. Wie im Ventil sind diese Magnete unterschiedlich gepolt. Bei dem einen Magneten liegt der Südpol unten, bei dem anderen liegt der Nordpol unten. Die Position der beiden Magnete auf der Achse korrespondiert mit der Position der Skalierung, die auf Teil 47 angebracht ist. Diese Skalierung ist ebenfalls fest mit der Achse 41 verbunden. Die Buchse 48 dient als Lagerung für die Achse 41. Die Buchse wird eingebracht durch O-Ringe, durch die die Buchse in der Hülse 45 fixiert wird. Eine zweite Lagerbuchse ist am oberen Bereich des Stiftes angebracht, Teil 42. Auch hier ist Achse 41 als Gleitlagerung in Buchse 42 fixiert. Der Verstellstift enthält zwei verschiedene Federn: eine starke Feder 44 und eine extrem weiche Feder 46. Durch Druck auf Knopf 39 wird die Achse 41, die im unteren Bereich eine kolbenartige Erweiterung hat, gegen die Federkraft 44 nach unten verschoben. Hierdurch wird die Achse 51 gegen die Federkraft der deutlich weicheren Feder 46 nach unten verschoben. Die Feder 46 wird also stark komprimiert, wo hingegen die Feder 44 nur wenig komprimiert wird. Die Kraft der Feder 44 wird durch die Achse 51 auf deren untere Spitze übertragen, die im Anwendungsfall die Kraft auf das zu entkuppelnde Ventil ausüben soll. Der Durchmesser der Achse an der Spitze sollte vorzugsweise etwa 3 mm betragen, das untere Ende sollte kuppelförmig abgerundet sein. Die im unteren Ende des Stiftes angebrachte Kappe 52 schützt die Lagerung sowie die in der Achse 41 eingebrachten Magnete 50. Durch das Fenster 53 ist die Stellung der Magnete über die Skala der Skalentrommel 47 ablesbar. Durch die vorgeschlagene Konstruktion ist es möglich, die Verstelleinheit denkbar klein zu bauen, ohne die Verstellsicherheit negativ zu beeinträchtigen. Erstmals wird es möglich, solche Verstellstifte zu realisieren. Die Konstruktion ermöglicht ein möglichst nahes Platzieren der Magnete auf der Haut des Patienten. Unter gleichzeitiger Druckbelastung des Ventilgehäuses kann eine feine und präzise Verstellung vorgenommen werden.

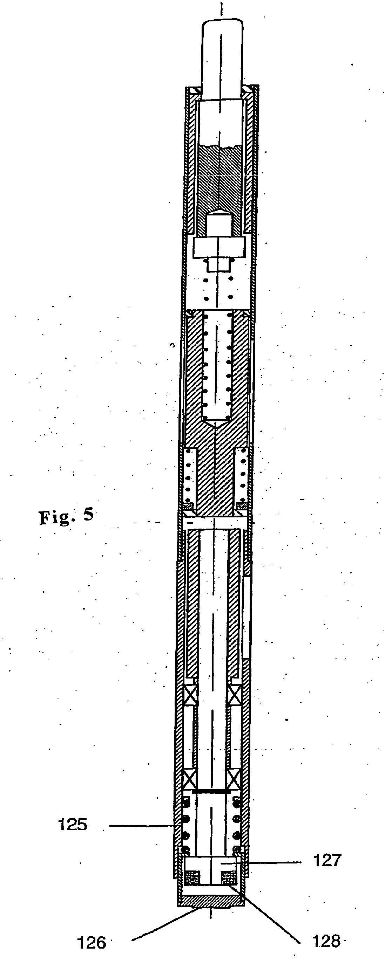

Zur Drehung des Formteiles dient eine Verstelleinrichtung, wie sie in Fig. 5 dargestellt ist. Zu der Verstelleinrichtung gehört ein Gehäuse 125 mit einer Kappe 126, mit der die Verstelleinrichtung auf dem Ventil aufgesetzt wird.

In dem Gehäuse 125 ist ein Kopf 127 mit zwei Stiftmagneten 128 vorgesehen. Die Stiftmagneten 128 besitzen den gleichen Abstand wie die Magneten des Rotors 1, sind aber so angeordnet, dass sie beim Aufsetzen der Verstelleinrichtung auf dem Ventil mit anderen Polen zu den Magneten des Formteiles weisen. Dadurch ziehen sich die Magnete an und folgt der Rotor 1 einer Drehung bzw. einer Schwenkbewegung der Verstelleinrichtung durch eine gleichsinnige Drehung bzw. gleichsinnige Schwenkung. Vorteilhafterweise erleichtert sich auch die genaue Positionierung der Verstelleinrichtung. Bei leichter Berührung führt die Anziehungskraft der Magneten die Verstelleinrichtung in die richtige Position.

Anschließend wird die Andrückung verstärkt, um eine geringfügige Verformung des Ventilgehäuses zu verursachen. Dabei wird der Gehäusedeckel elastisch verformt. Um die Verformung zu erleichtern, ist der Deckel 102 mit einer Verformungsdicke versehen. Die Verformungsdicke beträgt im Ausführungsbeispiel 0,2mm. Die Folge der Verformung ist ein Abheben des Rotors 1 von den zugehörigen Reibungsflächen. Die Reibung wird aufgehoben. Entsprechend leicht läßt sich der Rotor drehen oder schwenken.
Um der erfindungsgemäßen Verformung Rechnung zu tragen, ist in dem Gehäuse ein entsprechender Freiraum geschaffen. Ein vorteilhaftes Ausführungsbeispiel ist in den Fig. 8 bis 10 dargestellt. Darin ist ein scheibenförmiges Ventil mit einem ringförmigen Gehäuse 330 vorgesehen. Das Gehäuse 330 ist beidseitig mit Deckeln versehen. Die zu den Deckeln gehörenden Öffnungen im Gehäuse 330 erlauben die Montage aller innen im Ventil vorgesehenen Teile.

Das Gehäuse 330 ist mit einem Anschlußstutzen 302 und einem Abflußstutzen 312 versehen. Der Anschlußstutzen 302 ist an dem außenseitigen Ende als Anschluß für die Schlauchleitung ausgebildet. Innenseitig ragt der Anschlußstutzen durch die Gehäusewand 330 bis in den Gehäuseinnenraum, so dass im Gehäuseinnenraum eine entlang der Gehäuseinnenwand nach unten verlaufende Rohrleitung 314 an den Anschlußstutzen angeschlossen werden kann. Zugleich ist das innenseitige Ende des Anschlußstutzens 302 verschlossen, so dass der gesamte anströmende Liquor in die Rohrleitung 314 gezwungen wird.
Die Rohrleitung 314 führt zu dem Abflußstutzen 312, der in Teilen ähnlich wie der Anschlußstutzen 302 ausgebildet ist. Der Anschlußstutzen 312 ist in gleicher Weise montiert wie der Anschlußstutzen 302. Der Abflußstutzen 312 bildet im Unterschied zu dem Anschlußstutzen einen Ventilsitz für eine Ventilkugel 310. Der Ventilsitz hat die Form einer konischen Bohrung. Die Rohrleitung 314 ist an eine Zuführungsbohrung 309 im Abflußstutzen angeschlossen. Die Zuführungsbohrung 309 bildet eine Verbindung der Rohrleitung 314 zu der zusammen mit der Saphirkugel den Ventilsitz bildenden konischen Bohrung.

In der dargestellten Stehendlage des Ventils drückt die Ventilkugel 310 gegen den Ventilsitz. Außerdem drückt eine schwenkbeweglich im Ventilgehäuse angeordnete Kappe 308 auf die Ventilkugel 310. Infolgedessen muß die Liquorströmung Widerstand der Ventilkugel 310 und der Kappe 308 überwinden, um oben aus dem Abflußstutzen wieder auszutreten. Der oben austretende Liquor fließt in das Gehäuse 330 und von dort durch eine Öffnung 319, dann über die Austrittsöffnung 313 des Abflußstutzens 312 in einen nachfolgenden Teil der nicht dargestellten Schlauchleitung.

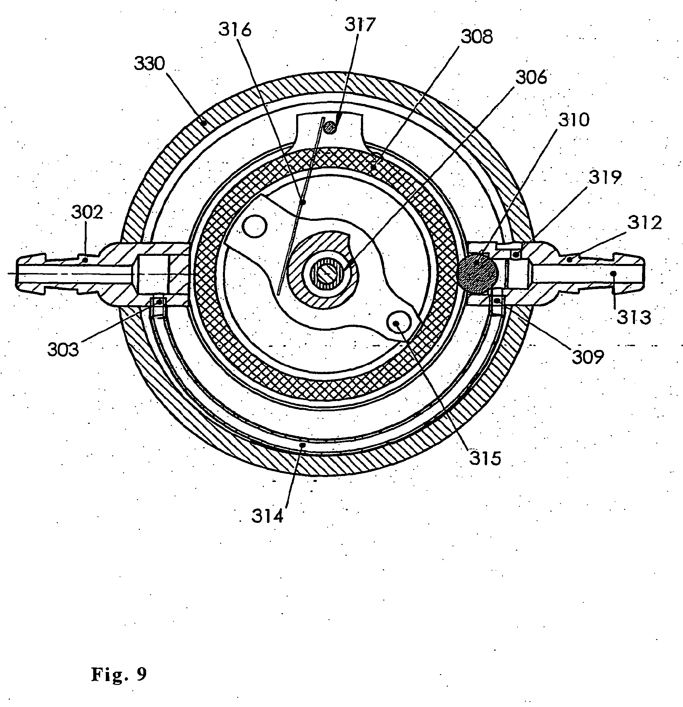

Aus Fig. 9 und 10 ist ersichtlich, dass die Kappe 308 aus einem zylindrischen Mantel besteht, der am Ende mit einem nach innen weisenden Kragen versehen ist. Die Kappe 308 umschließt einen Hohlraum, in dem im Ausführungsbeispiel eine Verstelleinrichtung angeordnet ist.
Die Kappe 308 besitzt in der Ansicht nach Fig. 9 rechts eine Lasche. In der Lasche befindet sich eine Bohrung für einen Lagerzapfen 317. Mit dem Lagerzapfen 317 ist die Kappe 308 verschweißt und schwenkbeweglich in dem Ventilgehäuse gelagert. An dem Lagerzapfen 317 ist ein Federdraht 316 befestigt, im Ausführungsbeispiel angeschweißt. Somit bilden die Kappe 308, die Feder 316 sowie der Lagerzapfen 317 eine formschlüssige Verbindung

Der Federdraht 316 ist durch eine Öffnung in dem zylindrischen Mantel der Kappe 308 in deren Hohlraum geführt. Die Öffnung besitzt eine Öffnungsweite, die für eine geringe Schwenkbewegung bzw. Verformung des Federdrahtes ausreicht.

Durch die Verformung des Federdrahtes wird ein Drehmoment an dem Lagerzapfen 317 erzeugt. Das Drehmoment wirkt über den Lagerzapfen 317 auf die Kappe 308 und in der Stehendlage dem Gewicht der Kappe 308 entgegen.

Die Verformung des Federdrahtes wird mittels eines Rotors 305 erzeugt. Der Rotor 305 ist drehbeweglich auf einer Achse 304 gelagert, der an einem Deckel 331 des Gehäuses befestigt ist. Der Deckel 331 ist nach außen gewölbt. Zugleich ist der auf dem Zapfen 304 sitzende Rotor 305 mit einer Schraube oder einem Klemmring 306 gegen den Deckel 331 verspannt. Dabei berührt der Rotor 305 mit seinen Außenkanten den Deckel 331. Die Verspannung hat den Vorteil, dass zum Lösen der Bremse bzw. zum Lösen der Arretierung ein Mindestdruck erforderlich ist, der höher ist als übliche Druckbelastungen aus dem täglichen Leben, z. B. der Druckbelastung aus einer Liegendstellung. Dies verhindert, dass sich der Rotor 305 unbeabsichtigt von dem Deckel 331 löst.
Im Übrigen reicht eine geringe Verformung des Deckels aus, um den Rotor 305 für eine Verstellung von dem Deckel zu lösen.

Die Verstellung erfolgt mit Hilfe von zwei Stiftmagneten 315, die in dem Rotor 305 montiert sind. Das geschieht wie in den zuvor erläuterten Ausführungsbeispielen mit anderen Magneten von außen (perkutan).

Der Federdraht 316 gleitet an einer kurvenförmig gestalteten Gleitfläche 333 des Rotors 305. Die Gleitfläche 333 ist im Ausführungsbeispiel so gestaltet, dass mit der Drehung des Rotors 305 eine gleichmäßige Abstandsänderung des aufliegenden Drahtendes zur Rotormitte stattfindet. Das ist im Ausführungsbeispiel gleichbedeutend mit einer gleichmäßigen Erhöhung oder Verringerung der Federkraft. In anderen Ausführungsbeispielen ist eine andere Abstandsänderung und Veränderung der Federkraft vorgesehen.

## Patentansprüche

1. Einstellbares Hydrocephalusventil zum Druckausgleich des Liquor im Schädel eines Hydrocephaluspatienten,
wobei das Ventil dem Patienten implantierbar ist und vorzugsweise über eine gleichfalls implantierbare Schlauchleitung der überschüssige Liquor aus den Hirnkammern im Schädel des Patienten abgeleitet und vorzugsweise in die obere Hohlvene oder in den Bauchraum drainiert werden kann,
wobei das Ventil ein Ventilgehäuse (16,125,330) mit einem darin angeordneten Schließteil umfaßt, das in der Schließstellung gegen die Ventilöffnung drückt,
wobei der Ventildruck zumindest in der Stehendposition des Patienten zumindest teilweise durch das Gewicht des beweglichen, vorzugsweise als Kugel, Ring oder Scheibe ausgebildeten Schließteiles bestimmt wird,
**dadurch gekennzeichnet,**
**daß** eine perkutan verstellbare Feder (4,10,30,44,46,202) in der Stehendposition das Gewicht des Schließteiles teilweise oder vollständig kompensiert.

2. Ventil nach Anspruch 1, wobei neben dem Schließteil noch ein Gravitationssteil in dem Ventilgehäuse (16,125,330) vorgesehen ist, daß in der Stehendposition eine kraftschlüssige Verbindung mit dem Schließteil hat, wobei die Feder(4,10,30,44,46,202) mit dem Gravitationsteil verbunden ist und verstellbar ist, so daß die Auflast des Gravitationsteiles auf dem Schließteil in der Stehendposition einstellbar ist,
**dadurch gekennzeichnet, daß** das Gravitationsteil als Scheibe oder Ring oder Kappe oder als Glocke ausgebildet ist und in dem Ventilgehäuse (16,125,330) schwenkbeweglich angeordnet ist.

3. Ventil nach Anspruch 1, **dadurch gekennzeichnet, daß** das Gravitationsteil durch mindestens eine zweite Kugel (204) gebildet wird, wobei die das Gravitationsteil bildende Kugel (204) im Falle der Verwendung einer Kugel (203) für das Schließteil in der Stehendlage über der Kugel (203) für das Schließteil angeordnet ist und die Feder (202) an die das Gravitationsteil bildende Kugel (204) angreift, vorzugsweise daß die Feder (202) zwischen die beiden Kugeln (203,204) grift.

4. Ventil nach Anspruch 1 oder 2, **dadurch gekennzeichnet, daß** die Verstelleinrichtung für die Feder (4,10,30,44,46) ganz oder teilweise innerhalb des Hohlraumes des Ringes oder Kappe oder Glocke angeordnet ist.

5. Ventil nach Anspruch 4, **dadurch gekennzeichnet, daß** das Schließteil des Ventils neben dem Ring oder Kappe oder Glocke angeordnet ist und daß die Feder (4,10,30,44,46) duch eine Öffnung in dem Ring oder Kappe oder Glocke zu dem Schließteil führt.

6. Ventil nach einem der Ansprüche 1,2,4,5, **dadurch gekennzeichnet, daß** das Gravitationsteil im Ventilgehäuse (16,125,330) geführt ist.

7. Ventil nach Anspruch 6, **dadurch gekennzeichnet, daß** die Führung durch eine schwenkbewegliche Lagerung gebildet wird oder eine Geradführung ist.

8. Ventil nach einem der Ansprüche 1 bis 7, **dadurch gekennzeichnet, daß** das Gravitationsteil ein größeres Gewicht als das Schließteil besitzt.

9. Ventil nach Anspruch 8, **dadurch gekennzeichnet, daß** für das Schließteil Rubin, Saphir, Titan und/oder für das Gravitationsteil Tantal Anwendung findet.

10. Ventil nach einem der Ansprüche 1,2,4 bis 9, **gekennzeichnet durch** eine Scheibenform des Ventils, wobei das Gravitationsteil mit einem für dessen Bewegung ausreichenden Abstand von der Gehäusewand bzw. dem Gehäuseboden oder Gehäusedeckel in dem Ventilgehäuse (16,125,330) und in einem für die Bewegung ausreichenden Abstand von den Einbauten im Ventilgehäuse (16,125,330) angeordnet ist.

11. Ventil nach einem der Ansprüche 1,2,4 bis 10, **dadurch gekennzeichnet, daß** das Ventilgehäuse (16,125,330) mindestens einen Deckel besitzt und die das Gravitationsteil bildende Scheibe oder Ring oder Kappe oder Glocke einen Durchmesser besitzt, der kleiner als die mit dem Deckel korrespondierende Öffnung im Ventilgehäuse (16,125,330) ist.

12. Ventil nach Anspruch 7, **dadurch gekennzeichnet, daß** die Feder (4,10,30,44,46,202) ganz oder teilweise an der Schwenkachse befestigt ist.

13. Ventil nach einem der Ansprüche 1 bis 12, **dadurch gekennzeichnet, daß** die Feder (4,10,30,44,46,202) als Federdraht oder als Blattfeder ausgebildet ist.

14. Ventil nach einem der Ansprüche 1 bis 13, **dadurch gekennzeichnet, daß** die Feder (4,10,30,44,46,202) mit einer Verstelleinrichtung versehen ist.

15. Ventil nach Anspruch 14, **dadurch gekennzeichnet, daß** die Verstelleinrichtung ein schwenkbewegliches oder drehbewegliches Teil besitzt, das von außen durch Schwenken oder Drehen mittels Magneten bewegt wird, so daß die Feder(4,10,30,44,46,202) gespannt oder entlastet wird.

16. Ventil nach Anspruch 14 oder 15, **gekennzeichnet durch** eine aktivierbare Bremse, **durch** deren Aktivierung eine nicht intendierte Verstellbewegung verhindert und **durch** deren Deaktivierung die Verstellbewegung ermöglicht wird.

17. Ventil nach Anspruch 16, **gekennzeichnet durch** eine selbstaktivierende Bremse, die **durch** Entlastung von dem Federdruck deaktiviert wird.

18. Ventil nach Anspruch 17, **dadurch gekennzeichnet, daß** das
a)Ventilgehäuse (16,125,330) nachgiebig ausgebildet ist und Reibungsflächen aufweist, die im unbelasteten Zustand des Ventilgehäuses (16,125,330) reibungsschlüssig an mindestens einem Teil der Verstelleinrichtung anliegen und sich durch Zusammendrückung des Ventilgehäuses (16,125,330) von den korrespondierenden Flächen der Verstelleinrichtung abheben oder
b)ein Ventilgehäuse (16,125,330) mit einem nachgiebigen Deckel versehen ist, der im unbelasteten Zustand reibungsschlüssig an mindestens einem Teil der Verstelleinrichtung anliegt und sich unter Druck von den korrespondierenden Flächen der Verstelleinrichtung abhebt.

19. Ventil nach Anspruch 18, **dadurch gekennzeichnet, daß** das nachgiebige Gehäuse (16,125,330) oder der Deckel
a)in der Arretierungsstellung der Bremse eine nach außen gewölbte Ausgangsform aufweist und nach dem Zusammendrücken eine verringerte Wölbung oder eine ebene Fläche oder eine Einwärtswölbung bildet.
b)in der Arretierungsstellung der Bremse eine ebene Ausgangsform aufweist und nach dem durch Zusammendrücken eine Einwärtswölbung aufweist.
c)in der Arretierungsstellung der Bremse eine Einwärtswölbung aufweist und nach dem Zusammendrücken eine vergrößerte Einwärtswölbung bildet oder die Gehäusewand eine einwärts gewölbte Ausgangsform besitzt und durch die Verformung eine weitere Einwärtswölbung erfährt.

20. Ventil nach einem der Ansprüche 17 bis 19, **gekennzeichnet durch** die Verwendung eines drehbeweglich oder schwenkbeweglich angeordneten Verstelltellers oder Rotors, der die Reibungsflächen für die Arretierung am äußeren Rand besitzt.

21. Ventil nach einem der Ansprüche 17 bis 20, **gekennzeichnet durch** die Verwendung eines drehbeweglich oder schwenkbeweglich angeordneten Verstelltellers oder Rotors, der mit der zusammendrückbaren Gehäusewand bzw. mit dem eindrückbaren Deckel vorgespannt ist.

22. Ventil nach Anspruch 21, **gekennzeichnet durch** eine Verformung der Gehäusewand für die Vorspannung bis zu einem Maß, daß gleich dem zweifachen der Gehäusewandstärke ist, vorzugsweise bis 0,1 mm.

23. Ventil nach einem der Ansprüche 17 bis 22, **dadurch gekennzeichnet, daß** ein Verstellteller verwendet wird, der im Querschnitt eine U-Form besitzt, so daß der Reibungsschluss an dem vorstehenden Rand der U-Form entsteht.

24. Ventil nach einem der Ansprüche 17 bis 23, **gekennzeichnet durch** Verwendung einer eindrückbaren Gehäusewand oder eines eindrückbaren Deckels mit einer Dicke bis 0,5mm, vorzugsweise mit einer Dicke bis 0,2 mm.

25. Ventil nach einem der Ansprüche 1 bis 24, **dadurch gekennzeichnet, daß** das Gehäuse (16,125,330), vorzugsweise die verformbare Gehäusewand, aus einem Metall besteht, vorzugsweise aus Titan bzw. einer Titanlegierung.

26. Ventil nach einem der Ansprüche 14 bis 25, **dadurch gekennzeichnet, daß** zu der Verstelleinrichtung ein perkutan anzudrückendes, drehbares Teil mit Verstellmagneten (50,128) gehört.

27. Ventil nach Anspruch 26, **gekennzeichnet durch** eine Drucksteuerung des drehbaren Teiles.

28. Ventil nach Anspruch 27, **gekennzeichnet durch** die Verwendung von Federgliedern für die Druckanzeige und die Druckbegrenzung und/oder die Verwendung von Dehnungsmeßstreifen für die Druckmessung.

29. Ventil nach einem der Ansprüche 14 bis 28, **gekennzeichnet durch** die Verwendung von Magneten(2,3,35,36,50,128,315) mit einem Durchmesser bis 3mm, vorzugsweise einem Durchmesser bis 1 mm und einer Höhe bis 5mm, vorzugsweise einer Höhe bis 2mm.

30. Ventil nach einem der Ansprüche 14 bis 29, **dadurch gekennzeichnet**, da die Maganete (2,3,35,36,50,128,315) in der außen liegenden Verstelleinrichtung einen Abstand voneinander aufweisen, der von den Magneten (2,3,35,36,50,128,315) in der innen liegenden Verstelleinrichtung höchtens um 3 mm, vorzugsweise höchstens um 1 mm abweicht und/oder daß die Magnete (2,3,35,36,50,128,315) höchstens einen Abstand von 20mm, vorzugsweise höchstens einen Abstand von 10mm und noch weitere bevorzugt höchsten einen Abstand von8 mm voneinander aufweisen.

31. Ventil nach einem der Ansprüche 14 bis 30, **dadurch gekennzeichnet, daß** die außen liegende Verstelleinrichtung mit einer Messeinrichtung für die Verstellbewegung versehen ist.

32. Ventil nach Anspruch 31, **dadurch gekennzeichnet, daß** die Messeinrichtung eine Druckmesseinrichtung und/oder eine Drehmesseinrichtung ist.

33. Ventil nach Anspruch 32, **dadurch gekennzeichnet daß** die außen liegende Verstelleinrichtung frei auf die Magnetstellung im Ventil einstellbar ist und daß die Drehstellung der Magnete (2,3,35,36,50, 128,315) außen ablesbar ist.

34. Ventil nach einem der Ansprüche 14 bis 33, **dadurch gekennzeichnet, daß** die innen liegende Verstelleinrichtung eine Feder (4,10,30,44,46,202) in der Form eines metallischen Drahtes oder Bleches besitzt, dessen Querschnitt vorzugsweise rund oder rechteckig ist und dessen Durchmesser oder Dicke bis 0,5mm, vorzugsweise bis 0,3 mm und noch weiter bevorzugt bis 0,2mm beträgt.

35. Ventil nach Anspruch 34, **dadurch gekennzeichnet, daß** der Federstab als
a)einarmiger Hebel ausgebildet ist oder
b)doppelarmiger Hebel ausgebildet ist, dessen einer Hebelarm mit dem Verstellteller und dessen anderer Hebelarm mit der Ventilkugel (5,105,203,204,310) oder Ventilklappe des Ventils drückt.

36. Ventil nach Anspruch 35, **dadurch gekennzeichnet, daß** der Federstab gelenkig gelagert ist und gleitend gegen eine Kurvenscheibe des Verstelltellers drückt und/oder gleitend gegen die Ventilkugel (5,105,203,204,310) oder Ventilklappe des Ventils drückt.

37. Ventil nach einem der Ansprüche 1 bis 36, **dadurch gekennzeichnet, daß** das Ventil einen Außendurchmesser bis 31mm, vorzugsweise bis 20mm und/oder eine Höhe bis 10mm, vorzugsweise bis 6mm aufweist.

38. Ventil nach einem der Ansprüche 1 bis 37, **dadurch gekennzeichnet, daß** das Gravitationsteil mit dem einzustellenden Federelement formschlüssig verbunden ist.

## Claims

1. Adjustable hydrocephalus valve to equalise the pressure of the fluid in the cranium of a hydrocephalus patient,
wherein the valve can be implanted in the patient and the excess fluid can be diverted from the ventricles of cerebrum in the cranium of the patient preferably by means of a likewise implantable flexible tube and preferably drained into the upper vena cava or into the abdominal cavity of the patient, wherein the valve includes a valve housing (16, 125, 330) in which is located a closing element that in the closed position presses against the valve opening,
wherein the valve pressure, at least in the upright position of the patient, is determined at least partially by the weight of the mobile closing element that is preferably in the form of a sphere, ring or disc, **characterised in that**
a percutaneously adjustable spring (4, 10, 30, 44, 46, 202) partially or totally compensates the weight of the closing element in the upright position.

2. Valve according to claim 1, wherein, in addition to the closing element, a gravitational element is provided in the valve housing (16, 125, 330), which in the upright position has a force-fit connection with the closing element, wherein the spring (4, 10, 30, 44, 46, 202) is connected with the gravitational element and is adjustable, such that the superimposed load of the gravitational element on the closing element is adjustable in the upright position,
**characterised in that** the gravitational element is in the form of a disc or ring or cap or as a bell and is arranged in a pivotably movable manner in the valve housing (16, 125, 330).

3. Valve according to claim 1, **characterised in that** the gravitational element is formed from at least one second sphere (204), wherein the sphere (204) that forms the gravitational element, in the case where a sphere (203) is used for the closing element, is arranged in the upright position above the sphere (203) for the closing element and the spring (202) engages the sphere (204) that forms the gravitational element, preferably that the spring (202) acts between both spheres (203, 204).

4. Valve according to claim 1 or 2, **characterised in that** the adjusting device for the spring (4, 10, 30, 44, 46) is partially or totally arranged inside the cavity of the ring or cap or bell.

5. Valve according to claim 4, **characterised in that** the closing element of the valve is arranged beside the ring or cap or bell and that the spring (4, 10, 30, 44, 46) leads through an opening in the ring or cap or bell to the closing element.

6. Valve according to one of claims 1, 2, 4, 5, **characterised in that** the gravitational element is guided in the valve housing (16, 125, 330).

7. Valve according to claim 6, **characterised in that** the guide is formed by a pivotably movable bearing or a straight guide.

8. Valve according to one of claims 1 to 7, **characterised in that** the gravitational element is heavier than the closing element.

9. Valve according to claim 8, **characterised in that** ruby, sapphire, titanium is used for the closing element and/or tantalum for the gravitational element.

10. Valve according to one of claims 1, 2, 4 to 7, **characterised by** a discoidal shape of the valve, wherein the gravitational element is arranged at a sufficient distance from the housing wall or from the housing floor or housing cover for movement in the valve housing (16, 125, 330) and at a sufficient distance from the fixtures in the valve housing (16, 125, 330) for movement.

11. Valve according to one of claims 1, 2, 4 to 10, **characterised in that** the valve housing (16, 125, 330) has at least one cover and the disc or ring or cap or bell that forms the gravitational element has a diameter that is smaller than the opening in the valve housing (16, 125, 330) that corresponds to the cover.

12. Valve according to claim 7, **characterised in that** the spring (4, 10, 30, 44, 46, 202) is completely or partially fixed to the swivelling axis.

13. Valve according to one of claims 1 to 12, **characterised in that** the spring (4, 10, 30, 44, 46, 202) is in the form of a wire spring or a leaf spring.

14. Valve according to one of claims 1 or 13, **characterised in that** the spring (4, 10, 30, 44, 46, 202) is provided with an adjustment device.

15. Valve according to claim 14, **characterised in that** the adjustment device possesses a pivotably movable or rotatably movable element that is moved from the exterior by pivoting or turning by means of magnets, such that the spring (4, 10, 30, 44, 46, 202) is wound up or released.

16. Valve according to claim 14 or 15, **characterised by** an activatable brake that when activated prevents an unintended adjustment movement and when deactivated allows the adjustment movement.

17. Valve according to claim 16, **characterised by** a self-activating brake that is deactivated by releasing the spring pressure.

18. Valve according to claim 17, **characterised in that** the
a) valve housing (16, 125, 330) is resilient and has friction surfaces that in the unstressed state of the valve housing (16, 125, 330) are friction-locked on at least one part of the adjustment device and by pressing together the valve housing (16, 125, 330) are raised up from the corresponding surfaces of the adjustment device or
b) a valve housing (16, 125, 330) is provided with a resilient cover that in the unstressed state remains friction-locked to at least one part of the adjustment device and under pressure is raised up from the corresponding surfaces of the adjustment device.

19. Valve according to claim 18, **characterised in that** the resilient housing (16, 125, 330) or the cover
a) in the locked position of the brake exhibits an outwardly curved initial shape and after being pressed together forms a reduced curvature or a flat surface or an inward curvature,
b) in the locked position of the brake exhibits a flat initial shape and after being pressed together exhibits an inward curvature,
c) in the locked position of the brake exhibits an inward curvature and after being pressed together forms a greater inward curvature or the housing wall possesses a curved initial shape and after the deformation undergoes a further inward curvature.

20. Valve according to one of claims 17 to 19, **characterised by** the use of a rotatably or pivotably movable adjustment plate or rotor that possesses friction surfaces for locking on the external edge.

21. Valve according to one of claims 17 to 20, **characterised by** the use of a rotatably or pivotably movably arranged adjustment plate or rotor that is prestressed with the housing walls that can be pressed together or with the compressible cover.

22. Valve according to claim 21, **characterised by** a deformation of the housing wall for prestressing to a degree that is equal to twice the wall thickness of the housing, preferably up to 0.1 mm.

23. Valve according to one of claims 17 to 22, **characterised in that** an adjustment plate is used that possesses a U shaped cross section, such that the friction locking results on the projecting edge of the U shape.

24. Valve according to one of claims 17 to 23, **characterised by** the use of a pressable housing wall or a pressable cover with a thickness up to 0.5 mm, preferably with a thickness up to 0.2 mm.

25. Valve according to one of claims 1 to 24, **characterised in that** the housing (16, 125, 330), preferably the deformable housing wall, consists of a metal, preferably of a titanium or a titanium alloy.

26. Valve according to one of claims 14 to 25, **characterised in that** a percutaneously pressable, rotatable part with adjusting magnets (50, 128) belong to the adjustment device.

27. Valve according to claim 26, **characterised by** a pressure control of the rotatable part.

28. Valve according to claim 27, **characterised by** the use of spring members to indicate the pressure and the pressure limitation and/or the use of elongating measurement strips for measuring the pressure.

29. Valve according to one of claims 14 to 28, **characterised by** the use of magnets (2, 3, 35, 36, 50, 128, 315) with a diameter of up to 3 mm, preferably a diameter of up to 1 mm and a height of up to 5 mm, preferably a height of up to 2 mm.

30. Valve according to one of claims 14 to 29, **characterised in that** the magnets (2, 3, 35, 36, 50, 128, 315) in the external adjustment device are at a distance from each other that differs from the distance between the magnets in the internal adjustment device by a maximum of 3 mm, preferably by a maximum of 1 mm and/or the magnets (2, 3, 35, 36, 50, 128, 315) are at a maximum distance of 20 mm from each other, preferably at a maximum distance of 10 mm from each other, and even more preferably at a maximum distance of 8 mm from each other.

31. Valve according to one of claims 14 to 30, **characterised in that** the external adjustment device is provided with a measuring device for the adjustment movement.

32. Valve according to claim 31, **characterised in that** the measuring device is a pressure measuring device and/or a rotation measuring device.

33. Valve according to claim 32, **characterised in that** the external adjustment device adjusts freely to the magnet position in the valve and that the rotational position of the magnets (2, 3, 35, 36, 50, 128, 315) can be read externally.

34. Valve according to one of claims 14 to 33, **characterised in that** the internal adjustment device possesses a spring (4, 10, 30, 44, 46, 202) in the form of a metallic wire or sheet, the cross section of which is round or rectangular, with a diameter or thickness of up to 0.5 mm, preferably up to 0.3 mm and even more preferably up to 0.2 mm.

35. Valve according to claim 34, **characterised in that** the spring bar is
a) realised as an armed lever arm or
b) realised as a two-armed lever arm, of which one lever arm presses against the adjustment plate and the other lever arm of which presses against the valve ball (5, 105, 203, 204, 310) or valve cap of the valve.

36. Valve according to claim 35, **characterised in that** the spring bar is flexibly mounted and presses in a sliding contact against a cam plate of the adjustment plate and/or presses in a sliding contact against the valve ball (5, 105, 203, 204, 310) or valve cap of the valve.

37. Valve according to one of claims 1 to 36, **characterised in that** the valve has an external diameter of up to 31 mm, preferably up to 20 mm and/or a height of up to 10 mm, preferably up to 6 mm.

38. Valve according to one of Claims 1 to 37, **characterised in that** the gravitational element is connected in a positive locking manner with the spring element that is to be employed.

## Revendications

1. Soupape réglable pour patient atteint d'hydrocéphalie, pour l'equilibrage de la pression du liquide céphalorachidien: dans le crâne d'un patient souffrant d'hydrocéphalie,
la soupape étant implantable chez le patient et, de préférence, par l'intermediaire d'une conduite en tuyau souple implantable également, l'excès de liquide céphalorachidien est dérivé des chambres cervicales dans le crâne du patient et, de préférence, peut être drainé dans la veine cave ou dans l'espace abdominal, sachant que la soupape comprend un boîtier de soupape (16, 125, 330) avec une partie de fermeture y étant disposée, qui, en position de fermeture, presse contre l'ouverture de soupape,
sachant que la pression de soupape est déterminée, au moins à la position debout du patient , au moins en partie par le poids de la partie de fermeture mobile réalisée de préférence sous la forme de billes, d'anneaux ou de disques,
**caractérisée en ce qu'**
un ressort (4, 10, 30, 44, 46, 202), réglable de façon percutanée à la position debout, compense, partiellement ou complètement, le poids de la partie de fermeture.

2. Soupape selon la revendication 1, sachant que, outre la partie de fermeture, encore une partie de gravitation est prévue dans le boîtier de soupape (16, 125, 330), en ce que, à la position debout, une liaison, opérant par interaction de forces, est établie avec la partie de fermeture, sachant que le ressort (4, 1.0, 30, 44, 46, 202) est relié à la partie de gravitation et est réglable de manière que la charge de la partie de gravitation sur la partie de fermeture, à la position debout, soit réglable, **caractérisée en ce que** la partie de gravitation est réalisée sous la forme de disque ou d'anneau ou de capuchon ou de cloche et est disposée avec une mobilité de pivotement dans le boîtier de soupape (16, 125, 330).

3. Soupape selon la revendication 1, **caractérisée en ce que** la partie de gravitation est formée par au moins une deuxième bille (204), la bille (204), formant la partie de gravitation en cas d'utilisation d'une bille (203) pour la partie de fermeture à la position debout, est disposée au-dessus de la bille (203) prévue pour la partie de fermeture, et le ressort (202) agit sur la bille (204) formant la partie de gravitation, de préférence **en ce que** le ressort (202) agit entre les deux billes (203, 204).

4. Soupape selon la revendication 1 ou 2, **caractérisée en ce que** le dispositif de réglage pour le ressort (4, 10, 30, 44, 46, 202) est disposé, en totalité ou partiellement, à l'intérieur de l'espace creux de l'anneau, ou du capuchon ou de la cloche.

5. Soupape selon la revendication 4, **caractérisée en ce que** la partie de fermeture de la soupape est disposée à côté de l'anneau ou du capuchon ou de la cloche, et **en ce que** le ressort (4, 10, 30, 44, 46) est guidé par une ouverture, ménagée dans l'anneau ou le capuchon ou la cloche, vers la partie de fermeture.

6. Soupape selon l'une des revendications 1, 2, 4, 5, **caractérisée en ce que** la partie de gravitation est guidée dans le boîtier de soupape (16, 125, 330).

7. Soupape selon la revendication 6, **caractérisée en ce que** le guidage est formé par un montage en palier à mobilité de pivotement, ou bien est un guidage rectiligne.

8. Soupape selon l'une des revendications 1 à 7, **caractérisée en ce que** la partie de gravitation présente un poids supérieur à celui de la partie de fermeture.

9. Soupape selon la revendication 8, **caractérisée en ce que**, pour la partie de fermeture, est appliqué un rubis, un saphir, du titane et/ou, pour la partie de gravitation, est appliqué du tantale.

10. Soupape selon l'une des revendications 1, 2, 4 à 9, **caractérisée par** une forme discoïde de la soupape, sachant que la partie de gravitation est disposée avec un espacement suffisant pour son déplacement vis-à-vis de la paroi de boîtier ou du fond de boîtier ou le couvercle de boîtier, à l'intérieur du boîtier de soupape (16, 125, 330), et sous un espacement, suffisant pour le déplacement, vis-à-vis des accessoires intérieurs dans le boîtier de soupape (16, 125, 330).

11. Soupape selon l'une des revendications 1, 2, 4 à 10, **caractérisée en ce que** le boîtier de soupape (16, 125, 330) comprend au moins un couvercle, et le disque ou l'anneau ou le capuchon ou la cloche formant la partie de gravitation présente un diamètre inférieur à l'ouverture, correspondant au couvercle, dans le boîtier de soupape (16, 125, 330).

12. Soupape selon la revendication 7, **caractérise en ce que** le ressort (4, 10, 30, 44, 46, 202) est fixé, en totalité ou en partie, sur l'axe de pivotement.

13. Soupape selon l'une des revendications 1 à 12, **caractérisée en ce que** le ressort (4, 10, 30, 44, 46, 202) est réalisé sous la forme de fil à ressort ou de ressort à lame.

14. Soupape selon l'une des revendications 1 à 13, **caractérisée en ce que** le ressort (4, 10, 30, 44, 46, 202) est muni d'un dispositif de réglage.

15. Soupape selon la revendication 14, **caractérisée en ce que** le dispositif de réglage comprend une partie à mobilité de pivotement, ou à mobilité de rotation, déplacée depuis l'extérieur par un pivotement ou une rotation effectuée au moyen d'aimants, de manière que le ressort (4, 10, 30, 44, 46, 202) soit tendu ou déchargé.

16. Soupape selon la revendication 14 ou 15, **caractérisée par** un frein activable, dont l'activation empêche un déplacement de manoeuvre escompté, et le déplacement de réglage est rendu possible par sa désactivation.

17. Soupape selon la revendication 16, **caractérisée par** un frein à auto-activation, désactivé par décharge par la pression élastique.

18. Soupape selon la revendication 17, **caractérisée en ce que**
a) le boîtier de soupape (16, 125, 330) est réalisé de façon à être déformable et présente des faces de frottement, qui, lorsque le boîtier de soupape (16, 125, 330) se trouve à l'état déchargé, s'appliquent, avec une liaison par friction, sur au moins une partie du dispositif de réglage et, par compression du boîtier de soupape (le, 125, 330), se soulèvent, des faces correspondantes du dispositif de réglage, ou
b) un boîtier de soupape (16, 125, 330) est muni d'un couvercle déformable, qui, à l'état non chargé, appuie avec une liaison par friction en au moins une partie du dispositif de réglage et se soulève sous l'effet de la pression, vis-à-vis des surfaces correspondantes du dispositif de réglage.

19. Soupape selon la revendication 18, **caractérisée en ce que** le boîtier de soupape (16, 125, 330) déformable, ou le couvercle
a) à la position de blocage du frein, présente une forme initiale incurvée vers l'extérieur et, après compression, forme un bombement diminué, ou bien une surface plane ou un bombement vers l'intérieur,
b) à la position de blocage du frein, présente une forme initiale plane et, après compression, présente un bombement orienté vers l'intérieur,
d) à la position de blocage du frein, forme un bombement orienté vers l'intérieur et, après compression, présente un bombement orienté vers l'intérieur, accru, ou la paroi de boîtier présente une forme initiale incurvée vers l'intérieur et subit un bombement vers l'intérieur supplémentaire, du fait de la déformation.

20. Soupape selon l'une des revendications 17 à 19, **caractérisée par** l'utilisation d'un plateau de réglage ou rotor, disposé avec une mobilité de rotation ou une mobilité de pivotement, comprenant les surfaces de friction pour le blocage sur le bord extérieur.

21. Soupape selon l'une des revendications 17 à 20, **caractérisée par** l'utilisation d'un plateau de réglage ou un rotor, disposé avec une mobilité de rotation ou une mobilité de pivotement, précontraint par la paroi de boîtier comprimable, ou par le couvercle enfoncé vers l'intérieur.

22. Soupape selon la revendication 21, **caractérisée par** une déformation de la paroi de boîtier, pour la précontrainte jusqu'à une dimension égale au double de l'épaisseur de paroi de boîtier, de préférence jusqu'à 0,1 mm.

23. Soupape selon l'une des revendications 17 à 22, **caractérisée en ce qu'**est utilisé un plateau de réglage ayant, observé en vue en coupe, une forme en U, de manière que la liaison à friction apparaisse sur le bord en saillie de la forme en U.

24. Soupape selon l'une des revendications 17 à 23, **caractérisée par** l'utilisation d'une paroi de boîtier susceptible d'être enfoncée, ou d'un couvercle susceptible d'être enfoncé, d'une épaisseur allant jusqu'à 0,5 mm, de préférence avec une épaisseur allant jusqu'à 0,2 mm.

25. Soupape selon l'une des revendications 1 à 24, **caractérisée en ce que** le boîtier (16, 125, 330), de préférence la paroi de boîtier déformable, est formé (e) d'un métal, de préférence de titane ou d'un alliage de titane.

26. Soupape selon l'une des revendications 14 à 25, **caractérisée en ce qu'**une partie rotative, munie d'aimants de manoeuvre (50, 128) pouvant être pressés de façon percutanée, appartient au dispositif de réglage.

27. Soupape selon la revendication 26, **caractérisée par** une commande de pression de la partie rotative.

28. Soupape selon la revendication 27, **caractérisée par** l'utilisation d'organes élastiques, pour l'indication de pression et la limitation de pression, et/ou par l'utilisation de jauges extensométriques pour la mesure de pression.

29. Soupape selon l'une des revendications 14 à 28, **caractérisée par** l'utilisation d'aimants (2, 3, 35, 36, 50, 128, 315), avec un diamètre allant jusqu'à 3 mm, de préférence un diamètre allant jusqu'à 1 mm, et une hauteur allant jusqu'à 5 mm, de préférence une hauteur allant jusqu'à 2 mm.

30. Soupape selon l'une des revendications 14 à 29, **caractérisée en ce que** les aimants (2, 3, 35, 36, 50, 128, 315), dans le dispositif de réglage extérieur, présentent, vis-à-vis des aimants (2, 3, 35, 36, 50, 128, 315) se trouvant dans la dispositif de réglage intérieur, un espacement mutuel qui diffère au plus de 3 mm, de préférence au plus de 1 mm et/ou **en ce que** les aimants (2, 3, 35, 36, 50, 128, 315) présentent au maximum un espacement de 20 mm, de préférence au maximum un espacement de 10 mm et, de manière encore mieux préférée, au maximum un espacement de 8 mm les uns des autres.

31. Soupape selon l'une des revendications 14 à 30, **caractérisé**é **en ce que** le dispositif de réglage extérieur est muni d'un dispositif de mesure pour le déplacement de réglage.

32. Soupape selon la revendication 31, **caractérisée en ce que** le dispositif de mesure est un dispositif de mesure de pression et/ou un dispositif de mesure de rotation.

33. Soupape selon la revendication 32, **caractérisée en ce que** le dispositif de réglage extérieur est réglable librement à la position magnétique dans la soupape, et en de que la position en rotation des aimants (2, 3, 35, 36, 50, 128, 315) est lisible extérieurement.

34. Soupape selon l'une des revendications 14 à 33, **caractérises en ce que** le dispositif de réglage intérieur présente un ressort (4, 10, 30, 44, 46, 202) ayant la forme d'un fil ou d'une tôle métallique, dont la section transversale, de préférence, est ronde ou rectangulaire, et dont le diamètre ou l'épaisseur va jusqu'à 0,5 mm, de préférence jusqu'à 0,3 mm et, de manière encore mieux proférée, jusqu'à 0,2 mm.

35. Soupape selon al revendication 34, **caractérisée en ce que** la barre élastique
a) est réalisée sous la forme de levier à un bras, ou
b) est réalisée sous la forme de levier à deux bras, dont un bras de levier presse avec le plateau de réglage, et dont l'autre bras de levier presse avec la bille de soupape (5, 105, 203, 204, 310) ou le clapet de soupape de la soupape.

36. Soupape selon la revendication 35, **caractérisée en ce que** la barre élastique est montée de façon articulée et presse en glissant contre un disque formant came du plateau de réglage et/ou presse en glissant contre la bille de soupape (5, 105, 203, 204, 310) ou le clapet de soupape de la soupape.

37. Soupape selon l'une des revendications 1 à 36, **caractérisée en ce que** la soupape présente un diamètre extérieur allant jusqu'à 31 mm, de préférence jusqu'à 20 mm et/ou une hauteur allant jusqu'à 10 mm, de préférence jusqu'à 6 mm.

38. Soupape selon l' une des revendications 1 à 37. **caractérisée en ce que** la partie de gravitation est reliée, par une liaison à ajustement de formes, à l'élément élastique à régler.
